# EUROPÄISCHE PATENTSCHRIFT

(11) **EP 1 071 517 B1**
(45) Veröffentlichungstag und Bekanntmachung des Hinweises auf die Patenterteilung: **02.04.2008**
(21) Anmeldenummer: 00912446.2
(22) Anmeldetag: 03.02.2000
(51) Int. Cl.: B05B 11/02

(54) **SPENDER FÜR FLIESSFÄHIGE MEDIEN**
DISPENSER FOR FLOWABLE MEDIA
DISTRIBUTEUR POUR MILIEUX COULANTS

(30) Priorität: 14.02.1999 DE 19905993; 21.05.1999 DE 29908923 U
(43) Veröffentlichungstag der Anmeldung: 31.01.2001
(73) Patentinhaber: Ing. Erich Pfeiffer GmbH, 78315 Radolfzell (DE)
(72) Erfinder: FUCHS, Karl-Heinz, D-78315 Radolfzell (DE); RITSCHE, Stefan, D-78315 Radolfzell (DE)
(74) Vertreter: Patentanwälte Ruff, Wilhelm, Beier, Dauster & Partner
(86) Internationale Anmeldenummer: PCT/EP2000/000851
(87) Internationale Veröffentlichungsnummer: WO 2000/047332

(56) Entgegenhaltungen:
- WO-A-98/30335
- DE-U- 29 601 047
- US-A- 4 623 337

## Beschreibung

Bei der Ausgabe, insbesondere der Zerstäubung von pharmazeutischen oder kosmetischen Produkten treten häufig Probleme auf, die in der Notwendigkeit genauester Dosierung liegen oder durch die Verderblichkeit oder Kontaminationsanfälligkeit der Produkte bei einmal geöffneten Medienbehältern, Verschmutzung oder Kontaminationsgefahren in den Leitungswegen etc. entstehen. Es sind daher Einmal-Spender entwickelt worden, die in einem zugleich den Pumpenzylinder für eine Schubkolbenpumpe bildenden Medienbehälter das Produkt enthalten, das nach dem erstmaligen Öffnen dieses Behälters, z.B. durch eine einen Kolbenstopfen durchstoßenden Nadel, den Inhalt in einem oder mehreren Hüben ausgeben. Ein solcher Zerstäuber ist in der WO 96/24439 (vgl. auch DE-U-29601047) beschrieben. Nach der Benutzung muß der gesamte Spender entsorgt werden.

Die WO-A-98/30335 (dem Oberbegriff des unabhängigen Anspruchs entsprechend) zeigt einen solchen Zerstäuber mit einer Sollbruchstellen enthaltenden Rastmechanik, die zwei aufeinanderfolgende Betätigungshübe voneinander abgrenzt und jeweils eine Mindestbetätigungskraft für die Zerstäubung durch Sollbruchstellen sicherstellt.

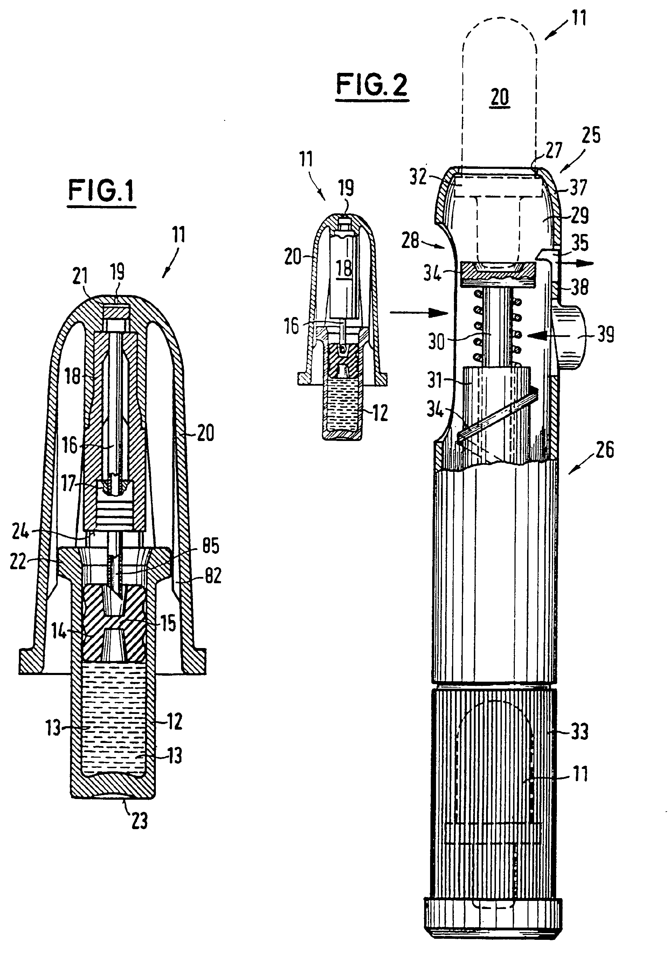

Die US-A-4623337 beschreibt einen Augentropfer mit einem Gehäuse und einer darin einsetzbaren Ampulle. Zur Betätigung ist ein Schraubgewinde in Verbindung mit einer Schrägflächen-Sprungmechanik vorgesehen. Die Ampulle enthält die Austragöffnung für die Augentropfen entweder selbst (Figur 1) oder es ist eine Hohlnadel vorgesehen, die einen Endverschluß der Ampulle durchstößt und die Austragöffnung bildet. Die mit dem Patienten und dem Medium in Kontakt kommenden Oberflächen des Gehäuses werden beim Auswechseln der Kartusche nicht mit ausgetauscht.

Aufgabe der Erfindung ist es, einen Spender zu schaffen, der Kontaminationen bei Mehrfach-Benutzung ausschließt und trotzdem eine Einsparung von Materialien ermöglicht. Dies wird durch die im Anspruch 1 definierte Lösung erreicht.

Insbesondere schafft die Erfindung einen Spender, der eine vom Benutzer leicht austauschbare Spendereinheit aufweist, die mit einer wiederverwendbaren Betätigungseinheit zur Benutzung verbunden und danach wieder getrennt werden kann, wobei die Betätigungseinheit für die weitere Benutzung wiederverwendet werden kann, während die Spendereinheit entsorgt wird. Die austauschbare Spendereinheit umfasst nicht nur, wie bei der DE 40 21 263 A, die Pumpenkammer und ihren Verschluß, sondern ermöglicht es, auch alle mit dem Medium in Berührung kommenden Teile austauschen zu können, also die Kanalwege, die Austrittsöffnung und ggf. die bedienungsnotwendig mit zu behandelnden Körperteilen in Berührung kommenden Elemente, nämlich eine sog. Nasenolive, d.h. einen Stutzen, der in die Nase eingeführt wird und der an seiner Spitze die Austrittsöffnung (Sprühdüse) enthält.

Die Erfindung zeigt zahlreiche Möglichkeiten auf, dies zu verwirklichen. Diese werden in der folgenden Beschreibung erläutert.

Die Zeichnungen zeigen bevorzugte Ausführungsbeispiele der Erfindung, die im Folgenden beschrieben werden. Es zeigen
- Fig. 1: einen Längsschnitt durch eine Spendereinheit,
- Fig. 2: einen Spender mit auslösbarer Federbetätigung im Teillängsschnitt und die zugehörige nachladbare Spendereinheit,
- Fig. 3: im Teillängsschnitt einen Spender und einen Längsschnitt durch die zugehörige austauschbare Spendereinheit,
- Figuren 4 und 5: Draufsichten auf zwei Versionen von Betätigungseinheiten,

- Fig. 6: in längsgeschnittener Explosionsdarstellung einen aus Spendereinheit und Betätigungseinheit bestehenden Spender mit einem Detail,
- Figuren 7 bis 11: jeweils im Längsschnitt Ausführungsformen von Spendern,
- Figuren 12 und 13: einen Teillängsschnitt und einen Längsschnitt in zwei senkrecht zueinander stehenden Ebenen durch einen Spender,
- Fig. 14: eine perspektivische Ansicht des Spenders,
- Figuren 15 bis 17: den Spender nach den Figuren 12 bis 14 in entsprechender Darstellungsweise in einer zweiten Arbeitsposition,
- Figuren 18 und 19: zwei Längsschnitte durch Ausführungsformen von Spendern,
- Fig. 20: eine Spendereinheit mit angedeuteter Befestigung an einer Betätigungseinheit,
- Fig. 21: die perspektivische Ansicht einer Schutzkappe,
- Figuren 22 bis 24: Längsschnitte durch nachladbare Spendereinheiten,
- Figuren 25 bis 27: schematische Darstellungen von Verriegelungsvorsprüngen und ihr Zusammenwirken mit der Betätigungseinheit,
- Fig. 28: einen Längsschnitt durch einen federbetätigten Spender,
- Fig. 29: die perspektivische Darstellung eines Auslöseelementes dafür,
- Fig. 30: eine schematische Darstellung der Wirkungsweise des Auslöseelementes,
- Fig. 31: einen Längsschnitt durch einen Spender,
- Figuren 32 und 33: in perspektivischer, teilgeschnittener Ansicht eine Ausführungsform einer Schutzkappe,
- Fig. 34: einen Längsschnitt durch die anhand von Fig. 32 und 33 erläuterte Schutzkappe mit der Spendereinheit,
- Fig. 35: einen Längsschnitt durch einen Spender,
- Figuren 36 und 37: Längsschnitte durch nachladbare Spendereinheiten,
- Fig. 38: einen Schnitt nach der Linie I-I in Fig. 37,
- Fig. 39: einen Längsschnitt durch eine Spendereinheit,
- Fig. 39a: ein Detail aus Fig. 39 in Draufsicht,
- Fig. 40: eine perspektivische Darstellung eines Spenders,
- Fig. 41: eine von einer Schutzkappe überdeckte Spendereinheit,

- Fig. 42: diese Spendereinheit im Längsschnitt und
- Fig. 43: einen Längsschnitt durch einen Spender mit Reserveaufnahme im Deckel.

Fig. 1 zeigt eine Bauart der Spendermechanik, wie sie bei den folgenden Ausführungsbeispielen Verwendung findet. Sie wird daher nur anhand von Fig. 1 detailliert beschrieben.

Bei der Ausführung nach Fig. 1 bildet sie eine Spendereinheit 11 mit einem Medienbehälter 12, der das auszugebende Medium 13 enthält, beispielsweise eine pharmazeutische oder kosmetische Flüssigkeit. Der Medienbehälter besteht zumeist aus Glas, wenn dies wegen der Diffusionssicherheit und Verträglichkeit des Materials erforderlich ist. Er bildet zugleich eine Schubkolben-Pumpenkammer, deren Kolben von einem Kolbenstopfen 14 gebildet wird, der die einzige Öffnung des Medienbehälters dicht verschließt. Er besteht aus einem sehr elastischen meist gummiartigen Kunststoffmaterial und hat eine relativ lange Mantelfläche, um eine sichere Führung bei seiner Kolbenfunktion zu haben. Umlaufende Wülste an der Mantelfläche sorgen für Dichtheit und kontrollierte Widerstandsverhältnisse bei Axialbewegung. In der Mitte ist durch zwei von beiden Seiten her vorgenommene zentrale Ausnehungen ein Durchstechbereich 15 mit geringerer Dicke abgegrenzt, der von einer Hohlnadel 16 ähnlich einer Injektionsnadel durchstochen werden kann. Diese ist, ggf. über eine aufgepreßte Haltebuchse 17, in einem Kolbenstößel 18 aufgenommen und reicht durch die mittlere Öffnung des Kolbenstößels 18 bis kurz vor eine Zerstäubungsdüse, die die Auslaßöffnung 19 bildet. Diese ist in einem Auslaßstutzen 20 vorgesehen, der als vorn abgerundeter Vorsprung den größten Teil der Spendereinheit ummantelt und den Kolbenstößel aufnimmt, der in ihn eingesetzt oder mit ihm einteilig verbunden ist. Der Kolbenstößel 18 grenzt mit dem Innenbereich der Austrittsöffnung eine Drallkammer 21 ab, die eine Zerstäubung in einem Sprühkegel ermöglicht, da die Flüssigkeit dort einem Drall ausgesetzt wird. Der Auslaßstutzen weist an seiner in Fig. 1 unteren, offenen Stirnfläche einen Außenflansch 32 auf.

Der Medienbehälter führt sich mittels seines im Öffnungsbereich vorgesehenen äußeren Flansches 22 in dem Inneren des Auslaßstutzens über innere Führungsstege 82.

### Die Spendereinheit arbeitet wie folgt:

Wenn der Medienbehälter 12, beispielsweise durch Druck auf seine Bodenfläche 23, in Richtung auf die Austrittsöffnung bewegt wird, durchsticht zuerst die Hohlnadel 16 den Durchstechbereich 15 des Kolbenstopfens 14. Kurz darauf erreicht die Stirnfläche 24 des Kolbenstößels 18 die obere Stirnfläche des Kolbenstopfens 14 und drückt diesen nach unten. Das Medium 13 tritt durch den Innenkanal 85 der Hohlnadel aus, der den Austrittskanal bildet und bis kurz vor die Austrittsöffnung 19 führt. Dort tritt die Flüssigkeit in die Drallkammer 21 ein und tritt danach, mit dem gehörigen Drall, aus der Austrittsdüse 19 als Sprühkegel in feinzerstäubter Form aus. Wenn der Kolbenstopfen 14 am Boden des Medienbehälters anschlägt, ist der Austragshub beendet. Es ist jedoch auch möglich, durch zwischengeschaltete Hindernisse, beispielsweise einen Zwischenanschlag, der überwindbar oder durch eine Sonderbetätigung zu umgehen ist, einen mehrstufigen Hub vorzusehen. Dies ist beispielsweise in der eingangs genannten WO 96/24439 beschrieben, auf die hier Bezug genommen wird. Alle Spendereinheiten, die im Rahmen dieser Beschreibung beschrieben sind, können mit Einmal- oder Mehrmalhub ausgebildet sein, wenn auch die Ausführung als Einmal-Zerstäuber bevorzugt ist.

Fig. 2 zeigt einen Spender 25, der eine Spendereinheit 11 gemäß Fig. 1 benutzt. Seine Betätigungseinheit 26 hat die Form eines langgestreckten zylindrischen Stifts, an dessen einer, in Fig. 2 oberen Stirnfläche eine Aufnahme- oder Ladeöffnung 27 für die Spendereinheit 11 vorgesehen ist. Sie wird durch einen Ladeausschnitt 28 in eine Ladekammer 29 eingesetzt, nachdem vorher ein Betätigungsstößel 30, der in einer Betätigungshülse 31 geführt ist, aus der Ladekammer nach unten zurückgezogen wurde. Die strichliert angedeutete Spendereinheit legt sich mit dem öffnungsseitigen Außenflansch 32 des Auslaßstutzens 20 im Randbereich der Aufnahmeöffnung 27 an.

In dem an die Ladekammer anschließenden Abschnitt der Betätigungseinheit 26 ist ein Drehmechanismus vorgesehen, der durch einen Drehknopf oder -knauf 33 am unteren Ende betätigt wird. Ähnlich wie bei einer Lippenstift-Mechanik wird die Betätigungshülse 30, z.B. über ein Steilgewinde 34, zurückgezogen oder in Richtung auf die eingesetzte Spendereinheit 11 bewegt. Wenn der Kopf 34 des BetätigungsstöBels 30 den Boden 23 des Medienbehälters 12 erreicht, wird dieser durch eine entsprechende Ausnehmung des Kopfes geführt. Gleichzeitig wird jedoch durch eine Auslöserklinke 35 der Betätigungsstö-Bel in der in Fig. 2 gezeigten Lage festgelegt. Die weiter aufwärtsschiebende Hülse 31 drückt eine Feder 36 zusammen und spannt diese für einen späteren Betätigungshub vor. Diese Position ist in Fig. 2 dargestellt.

Die Betätigungsklinke ist aus der Wandung des Gehäuses 37 der Betätigungseinheit durch entsprechende U-förmige Ausschnitte freigelegt, wobei seitliche Verbindungsstege 38 eine Art federndes Drehgelenk bilden. Dadurch bildet die Auslöseklinke zusammen mit einem auf der anderen Seite des Verbindungssteges 38 vorgesehenen, ebenfalls aus der Wandung durch einen U-Ausschnitt abgetrennten Auslöseknopf einen doppelarmigen Hebel.

Drückt der Benutzer auf den Auslöseknopf 39, so schwenkt dieser nach innen und die Auslöseklinke 35 nach außen (s. Pfeile). Der Betätigungsstößel 30 wird freigegeben, die Feder 36 drückt den Medienbehälter 12 nach oben und bewirkt so den Austraghub.

Nach der Betätigung wird durch entsprechende Drehung des Drehknopfes 33 der Betätigungsstößel und die Betätigungshülse wieder zurückgefahren, so daß die Ladekammer frei wird. Die verbrauchte Spendereinheit kann dann durch die Ladeöffnung 28 entnommen und durch eine neue ersetzt werden, was in Fig. 2 angedeutet ist. Es ist ebenfalls gezeigt, daß z.B. im Drehknopf 33 eine Reservekammer für eine Spendereinheit 11 gebildet sein kann.

Es sind zwar aus der DE 40 15 367 A schon Spender bekanntgeworden, die mit Federvorspannung und Auslösung arbeiten, aber hier wurde ein sehr attraktives Gerät mit einem hohen Grad an Bedienungsautomatik geschaffen, obwohl die Spendereinheit eine Einweg-Einheit ist. Dies ist besonders wichtig für Medikamente gegen stark schmerzhafte Krankheitserscheinungen, wie beispielsweise Migräne, wo ein entsprechendes, über die Haut (Nasenschleimhaut) aufzunehmendes Präparat beim Patienten immer in gebrauchsfähiger Form vorhanden sein sollte und von ihm auch besonders leicht zu verabreichen sein sollte, weil er durch die starken Schmerzen schon funktionsbehindert sein kann. Der Spender ist in Form des Stiftes besonders leicht mitzuführen und zu handhaben, wobei er nicht viel größer zu sein braucht als ein Füllfederhalter. Die Betätigung der Pumpe durch Federkraft sorgt für eine vorgegebene Betätigungskraft.

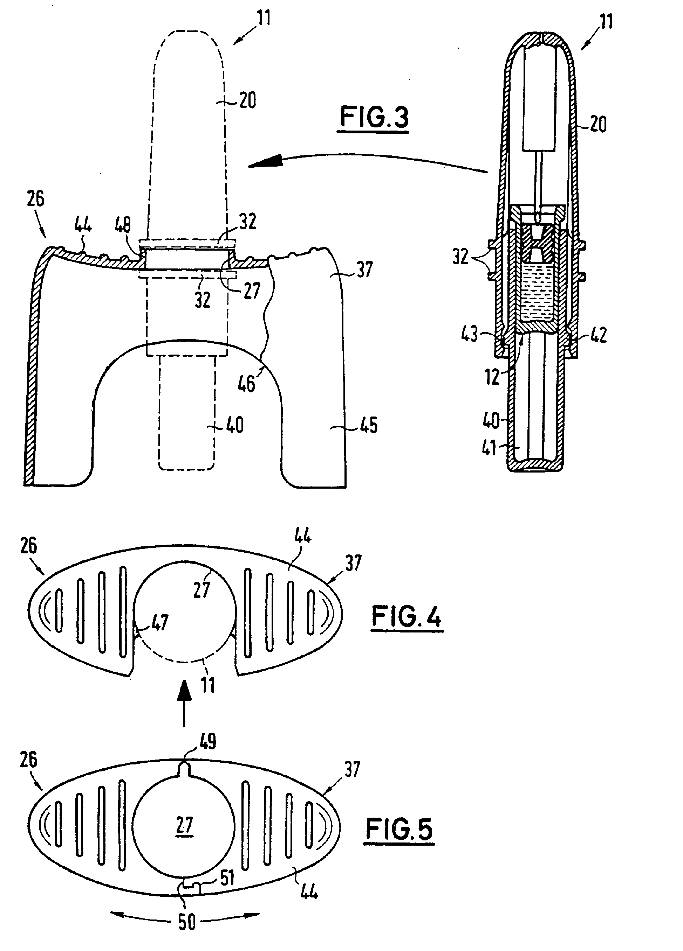

Fig. 3 zeigt eine besonders einfache, leicht herstellbare Ausführung, bei der eine Spendereinheit 11 vorgesehen ist, die zwei Außenflanschen 32 an dem Auslaßstutzen 20 aufweist. Der Medienbehälter 12 ist in einer Aufnahmehülse 40 aufgenommen, der, wie alle Teile der hier beschriebenen Zerstäuber, bis auf die Ampulle, die Hohlnadel und Federn, aus Kunststoffspritzguß bestehen kann.

Diese Hülse 40, in deren Inneren sich der Medienbehälter auf Stegen 41 abstützt, hat an ihrer Außenseite einen Ringvorsprung oder Flansch 42, der mit einer Ausnehmung 43 am Innenumfang des Auslaßstutzens 20 zusammenwirkt. Beide bilden eine durch Axialdruck überwindbare Rastverbindung, die, durch eine in Fig. 3 erkennbare einseitige, widerhakenartige Abschrägung des Flansches 42 an der Oberseite, leichter in Zusammendrückrichtung als in der Gegenrichtung zu überwinden ist. Es wird damit ein Betätigungsdruckpunkt geschaffen, der zu seiner Überwindung eine bestimmte Betätigungs-Mindestkraft erfordert. Es wird damit sichergestellt, daß die Betätigung zügig und ohne einen eventuellen Zwischenhalt erfolgt, wodurch die Zerstäubung beeinträchtigt werden könnte. Ein solcher Druckpunkt, dessen Funktion im einzelnen in der WO 96/24439 erläutert ist, ist bei allen im folgenden beschriebenen Versionen vorgesehen.

Fig. 3 zeigt, daß die Spendereinheit 11 in eine Betätigungseinheit 26 eingesetzt werden kann. Diese besteht im vorliegenden Fall aus einem Basisgehäuseteil 37, das eine flachovale Form hat (s. Fig. 4 und 5). An eine Ladeöffnung 27 schlie-Ben sich seitlich Betätigungsschultern oder -flächen 44 an.

Von diesen läuft ein ovaler Mantel 45 um, der jedoch in seinen beiden flacheren Seitenflächen Fingerausschnitte 46 hat. Dadurch ist der in diesem Teil liegende Abschnitt der Aufnahme- und Betätigungshülse 40 zwar vor versehentlicher Betätigung geschützt, jedoch für einen Daumen zur Betätigung zugänglich.

Fig. 4 und 5 zeigen zwei Möglichkeiten der Zusammenfügung der Spendereinheit mit der Betätigungseinheit 26. Nach Fig. 4 wird die Spendereinheit 11 in die in diesem Falle seitlich offene Ladeöffnung 27 hineingeschoben, und zwar mit dem zwischen den beiden Außenflanschen 32 liegenden Abschnitt. Rastvorsprünge 47 sichern die Spendereinheit gegen Herausfallen, während die beiden Außenflansche 32 zusammen mit einem Hals 48 der Ladeöffnung für eine Axialsicherung sorgen.

Nach Fig. 5 ist das Gehäuse 37 der Betätigungseinheit 26 aufklappbar. Es ist dabei zwar als einteiliges Kunststoffspritzgußteil hergestellt, jedoch über ein Filmscharnier 49 und eine Trennstelle 50 aufklappbar (s. Pfeile in Fig. 5). Die Spendereinheit 11 wird in die Ladeöffnung 28 im geöffneten Zustand des Gehäuses 37 eingefügt und dann dieses wieder geschlossen, wobei an der Trennstelle 50 eine Rastverbindung 51 vorgesehen ist.

Die Betätigung erfolgt manuell dadurch, daß der Bedienende je einen Finger auf je eine der Betätigungsschultern zu beiden Seiten des Auslaßstutzens 20 legt und mit dem Daumen die Aufnahmehülse 40 nach oben drückt.

Danach kann durch Ausrasten (Fig. 4) bzw. Aufklappen des Gehäuses (Fig. 5) die Spendereinheit ausgeworfen und entsorgt werden. Der weiterverwendete Teil, also die Betätigungseinheit 26, ist zwar hier als nur ein einstückiges Kunststoffteil gezeigt, ist jedoch das flächen- und ggf. auch volumenmäßig größte Teil, so daß sich seine Weiterverwendung lohnt. Ferner wird auch das Pack- und Transportvolumen einer mehrere Spendereinheiten umfassenden Behandlungseinheit wesentlich geringer, was insbesondere für Patienten wichtig ist, die stets mehrere Spendereinheiten mit sich führen müssen.

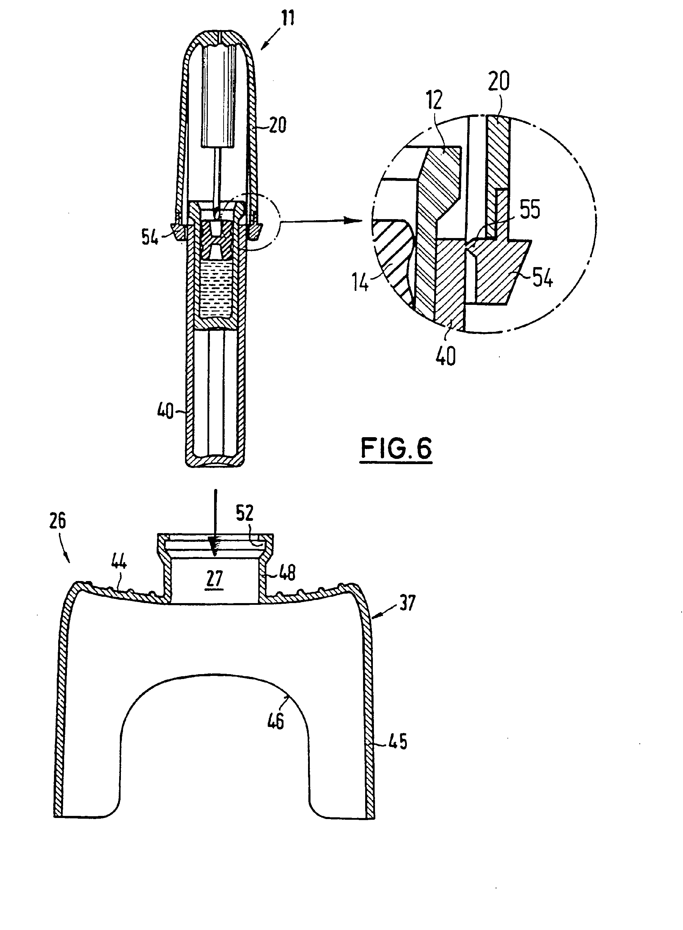

Fig. 6 zeigt eine ähnliche Ausführung, bei der die Betätigungseinheit 26 und ihr Gehäuse 37 der nach den Fig. 3 bis 5 in der Grundform entspricht. Hier ist jedoch die Ladeöffnung 27 in Form eines längeren Halses 48 ausgeführt, an dessen Oberseite eine umlaufende Rastnut 52 innen vorgesehen ist. Sie arbeitet mit einem entsprechenden schrägen Rastvorsprung 53 an der Spendereinheit 11 zusammen. Dieser ist an einem Ring 54 außen vorgesehen, der mit der Aufnahmehülse 40 einstückig gespritzt, jedoch nur über dünne Materialbrücken 55 verbunden ist. Diese bilden eine Sollbruchstelle, die die vorher beschriebene Druckpunktfunktion ebenso wie eine Originalitätssicherung schaffen. Der Ring ist von unten in eine Ausnehmung in der unteren Stirnfläche des Auslaßstutzens 20 eingesetzt (s. Detail bei Fig. 6).

Spendereinheit und Betätigungseinheit 11, 26 werden, wie in Fig. 6 gezeigt, zusammengesetzt. Die Spendereinheit wird mit ihrer Aufnahmehülse 40 zuerst von oben in die Ladeöffnung 27 eingeführt. Die Rastvorsprünge 53 rasten in die Rastausnehmungen 52 ein und legen die Spendereinheit dort fest.

Durch Druck mit dem Daumen auf die Bodenfläche der Aufnahmehülse 40 wird der Spender betätigt. Wie im Falle von Fig. 3 muß dabei zuerst der Druckpunkt überwunden werden (bei Fig. 3 durch Ausrasten der Schnappverbindung 42, 43 und bei Fig. 6 durch Zerstörung der eine Sollbruchstelle bildenden Materialbrücken 55). Mit der dadurch vorgegebenen Mindest-Betätigungskraft wird nun der Spender betätigt. Nach der Benutzung kann je nach Ausbildung der Rastausnehmungen und Vorsprünge die Spendereinheit entweder wieder nach oben entnommen oder auch nach unten durchgedrückt werden, um die Betätigungseinheit 26 für eine neue Charge freizumachen.

Statt der beschriebenen Rastverbindung ist auch eine Arretierung durch eine Art Bejonettverschluß zwischen Spendereinheit und Betätigungseinheit möglich. Ähnliches wird auch im folgenden noch beschrieben. Es ist zu erkennen, daß durch den relativ hohen Hals 48 der Auslaßstutzen 20 relativ kurz ausgebildet sein kann. Er kann auf den tatsächlich dem Körperkontakt ausgesetzten Teil beschränkt werden, während derjenige Teil, der zwischen den beiden Fingern des Benutzers liegt, am wiederverwendbaren Gehäuse 37 der Betätigungseinheit 26 bleibt. Dadurch wird Material an der Spendereinheit eingespart.

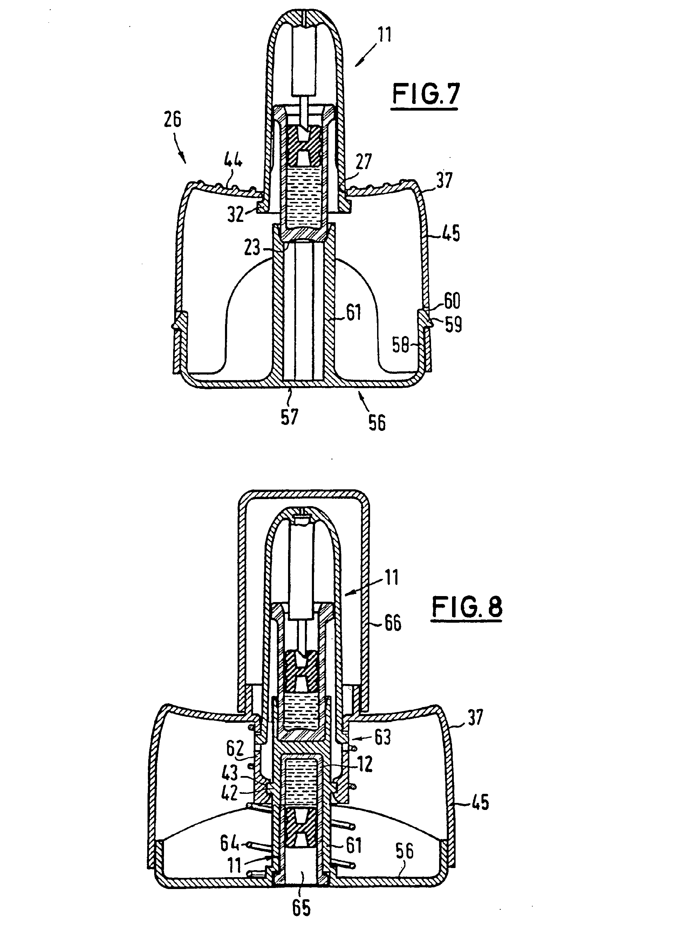

Bei der Ausführung nach Fig. 7 findet eine Spendereinheit nach Fig. 1, d.h. ohne Aufnahmehülse 40, Verwendung. Sie wird in die Ladeöffnung 28 des Gehäuses 37 der Betätigungseinheit 26 entweder durch einen Bajonettverschluß oder von unten her eingesetzt. In der ovalen unteren Öffnung des Gehäuses 37 ist ein Drücker 56 angeordnet, der diese untere Öffnung weitgehend überdeckt und mit der unteren Fläche 57 eine Betätigungsfläche bildet. Er führt sich mit seitlichen aufwärts gerichteten Wandungsteilen 58 innerhalb des Mantels 45 und greift mit oben abgeschrägten Vorsprüngen 59 in Öffnungen 60 im Mantel 45 ein. Ein ihrer Funktion der Aufnahmehülse 40 entsprechender hohler Mittelstutzen 61 stützt den Boden 20 des Medienbehälters 12.

Zum Einsetzen der Spendereinheit wird diese, bei Ausbildung mit Bajonettverschluß, von oben her in die Ladeöffnung 27 eingeführt und durch Drehen verriegelt. Der Medienbehälter liegt auf dem Aufnahmestutzen 61 auf und zentriert sich daran mittels einer konischen Aufnahmeöffnung.

Zur Betätigung wird auf die Bodenfläche 57, gedrückt, bis die Rasten 59, 60 den Drücker zur Bewegung nach oben freigeben. Der Aufnahmestutzen 61 drückt den Medienbehälter 12 nach oben und betätigt den Spender. Die Spendereinheit 11 kann dann mittels des Bajonettverschlusses nach oben wieder entnommen werden.

Es ist hier aber auch möglich, die Rastverbindung 59, 60 so auszubilden, daß die Rast 59 zum Auswerfen, beispielsweise durch Druck auf die beiden Flachseiten des ovalen Gehäuses 37 aus der Öffnung 60 freikommt, so daß der Drücker 56 nach unten herausgezogen werden kann. In diesem Falle brauchte der Auslaßstutzen 20 der Spendereinheit 11 nur den in Fig. 1 dargestellten Außenflansch 32 zur Anlage an der Unterkante der Ladeöffnung 28. Man könnte die Spendereinheit dann von unten her zusammen mit dem Drücker 56 einsetzen und nach dort wieder entnehmen.

Es ist auch möglich, im Innenräüm der Betätigungseinheit, d.h. zwischen den Betätigungsschultern 44 und der Innenfläche des Drückerbodens 57, eine Feder vorzusehen, die den Drücker 56 nach der Betätigung wieder in die Ausgangslage zurückführt.

Ähnlich ist dies in Fig. 8 gezeigt. Dort ist statt der Außenrasten 59, 60 in Fig. 7 eine druckpunktbildende Verrastung 42, 43 zwischen dem Aufnahmestutzen 61 des Drückers und einem inneren, den Aufnahmestutzen 20 umgebenden Stutzen 62 des Gehäuses 37 vorgesehen. Dieser Stutzen könnte auch nur durch Stege oder Finger gebildet sein, die die federnde Rastfunktion noch unterstützen würden. Die Spendereinheit 11 ist mittels einer Bajonettverbindung 62 eingesetzt, und zwar von oben her. Eine Feder 64 sorgt für die Rückholung in die in Fig. 8 dargestellte Ausgangslage nach der Betätigung.

In dem Aufnahmestutzen 61 ist eine Reservekammer 65 für eine Spendereinheit 11 vorgesehen. Bei dieser Ausführung könnte dann lediglich der Medienbehälter mit seinem Kolbenstopfen 14 ausgetauscht werden, nachdem der Auslaßstutzen 20 zusammen mit der Nadel, dem Kolbenstopfen und der Austrittsöffnung per Bajonett abgenommen wurde. Eine solche Ausführung ist dann sinnvoll, wenn eine Kontamination oder Verschmutzung der Austrittswege des Mediums nicht zu befürchten oder unbedenklich ist. Auch bei der Ausführung nach Fig. 7 wäre dies möglich. Die Rastverbindung 42, 43 sorgt für die Druckpunktfunktion und den Halt der Drückereinheit am Gehäuse 37.

Fig. 8 zeigt ferner eine Schutzkappe 66, die mit dafür sorgen kann, daß die Austrittswege für das Medium vor Kontamination, Verschmutzung und Austrocknung bewahrt werden, so daß dieser Teil der Spendereinheit zur wiederverwendbaren Betätigungseinheit gehören könnte.

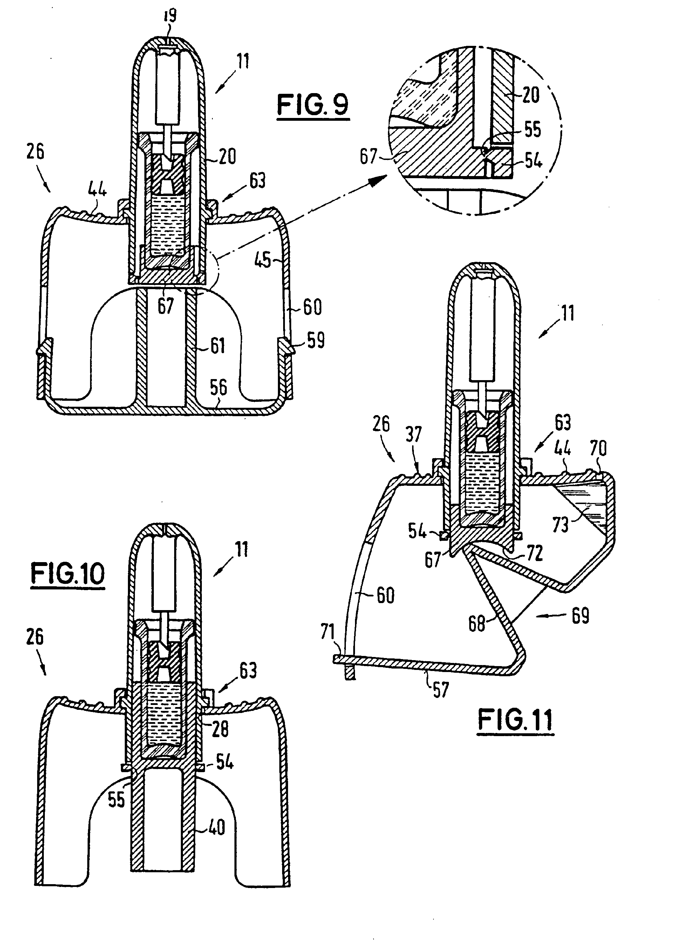

Fig. 9 zeigt eine Ausführung, bei der bei im übrigen ähnlichem Aufbau wie Fig. 7 die per Bajonett 63 eingesetzte Spendereinheit 11 an ihrer Unterseite durch eine Produktschutzkappe 67 verschlossen ist. Sie liegt am Boden des Medienbehälters 12 an, führt sich im Inneren des Auslaßstutzens und liegt auf seinem Unterrand mit einem Ring 54 auf (s. Detail), der über zerstörbare Materialbrücken 55 an die Produktschutzkappe 67 angespritzt ist. Der Drücker 56 ist über die widerhakenartig ausgeführten Rastvorsprünge 59 in der Öffnung 60 festgelegt, die jedoch schlitzförmig nach oben im Mantel 45 reicht, so daß sie eine Aufwärtsbewegung des Drückers 56 nicht behindert und nicht die Funktion des Betätigungsdruckpunktes übernimmt. Diese wird vielmehr von den Materialbrücken 55 übernommen, die bei der Betätigung zerstört werden, wenn der Mittelstutzen 61 des Drückers gegen den Boden der Produktschutzkappe 67 drückt und unter Zwischenschaltung dieser Kappe den Spender betätigt.

Diese Ausführung hat den Vorteil, daß die Spendereinheit 11 eine völlig geschlossene Einheit bildet, da die bis über den Medienbehälter heruntergezogene Wandung des Auslaßstutzens 20, die bis ins Gehäuse 37 hineinragt, und die die untere Öffnung verschließende Produktschutzkappe 67 den Medienbehälter und seine Kanalwege gänzlich umgeben. Wenn beispielsweise die Austrittsöffnung 19 noch mit einem Abreiß- oder Klebeverschluß versehen ist, dann kann auch beim Handhaben der Spendereinheit keine Kontamination eintreten.

Fig. 10 zeigt eine Ausführung, bei der die über ein Bajonett 63 in die Ladeöffnung 27 des Gehäuses 37 eingesetzte Spendereinheit 11 mit einer Aufnahmehülse 40 versehen ist, die den Sollbruchring 54 an ihrer Außenseite über die entsprechenden Materialbrücken 55 angespritzt aufweist. Er wirkt mit der ins Gehäuseinnere hinein verlängerten Auslaßstutzen-Stirnfläche zusammen, wenn auf die untere Stirnfläche der Aufnahmehülse 40 gedrückt wird. Der Ring 54 reißt dann ab und gibt den Hub mit der vorgesehenen Mindestbetätigungskraft frei.

Fig. 11 zeigt eine Ausführung, bei der die Spendereinheit 11 der nach Fig. 9 entspricht und auch über ein Bajonett 63 in das Gehäuse 37 der Betätigungseinheit 26 eingesetzt ist. Die untere Fläche der Produktschutzkappe 67 hat jedoch eine ggf. kugelkalottenartige Form, auf die ein Betätigungsstößel 68 des Gehäuses drückt. Dieser ist Teil eines Betätigungshebels 69, der an die Betätigungsschultern 44 einseitig über ein Filmscharnier 70 angespritzt ist. Er führt sich mit seinem äußeren Hebelende 71 in einem Schlitz 60 des Mantels 45 des Gehäuses 37. Wenn auf die untere Betätigungsfläche 57 gedrückt wird, dann verschwenkt sich der Hebel um sein Filmscharnier 70 im Uhrzeigersinn, und der Betätigungsstößel 68 drückt gegen die Kugelkalottenfläche 72 der Produktschutzkappe 67, reißt den Sollbruchring 54 ab und betätigt den Spender. Ein an den Hebelabschnitt 69 angespritzter Kunststofflappen 73 blockiert das Filmscharnier in der durch das Hebelende 71 und den Schlitz 60 gezeigten Position, so daß der Hebelabschnitt eine ggf. vorgespannte, mit dem Gehäuse 37 einstückige Kunststoff-Feder bildet. Mit dieser Ausführung kann über eine gewisse Hebelwirkung und durch federnde Ausbildung des Hebels 69 eine besonders effektive und schlagartige Betätigung des Spenders sowie eine Rückholung erreicht werden.

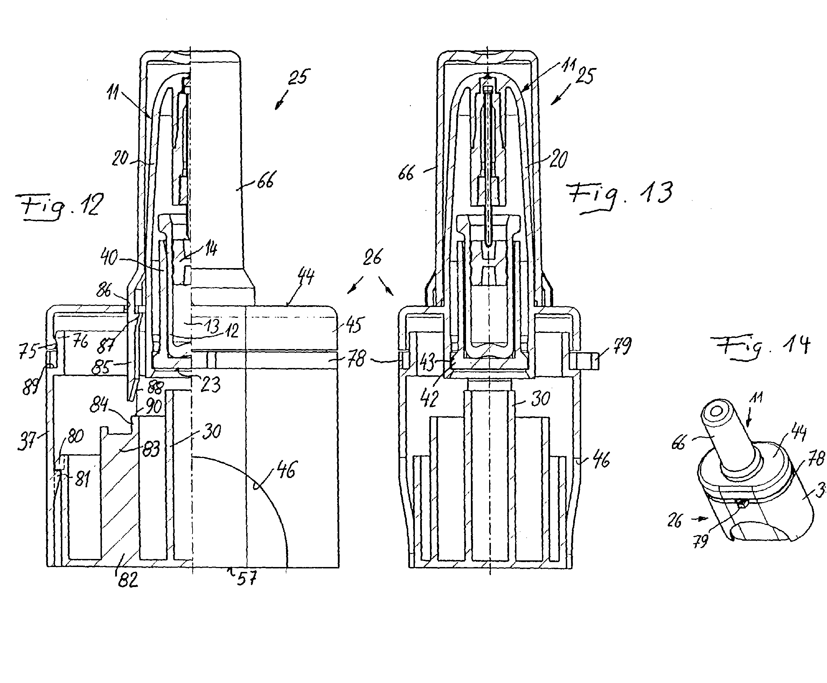

Eine Ausführung nach den Figuren 12 bis 17 zeigt einen Spender 25, dessen Spendereinheit 11, an dem Auslaßstutzen 20 angeformt, die Betätigungsschultern 44 aufweist, die nach Art einer ovalen, umgekehrten Pfanne ausgebildet sind und nur einen relativ kurzen, abwärtsgerichteten Mantel 45 aufweisen. Dieser hat an der Innenseite umlaufende Rastvorsprünge 75, die mit entsprechenden Vorsprüngen 76 an der Betätigungseinheit 26 zusammenwirken. Die Betätigungsschultern greifen so über den im Querschnitt ebenfalls oval geformten Körper 37 der Betätigungseinheit 26, daß eine durchgehende glatte Wand in Fortsetzung des Mantels 45 entsteht. Dabei ist jedoch, anschließend an den Mantel 45, ein Sicherungsring 78 zwischengelegt, der entweder als gesondertes Teil ausgebildet oder, bevorzugt, über abreißbare Materialbrücken an den Mantel 45 angeformt sein kann. Er hat eine Abreißlasche 79 und ist an dieser Stelle, ebenfalls über zerstörbare Materialbrücken, trennbar.

Körper oder Basisgehäuse 37 der Betätigungseinheit 26 sind ähnlich einem hohlen Rohr von ovalem Querschnitt ausgebildet. Über zusammenwirkende Rastnasen 80, 81 ist darin ein Betätigungskörper bzw. -drücker 82 gehalten, der in seinem Querschnitt im Innenquerschnitt des Körpers 37 entspricht und an seiner Unterseite eine Betätigungsfläche 57 aufweist, die über den Pumpenhub durch einen Betätigungsausschnitt 46 im Körper 37 für den Daumen des Benutzers zugänglich ist.

Der Betätigungskörper 82 enthält in seiner Mitte einen beispielsweise rohrförmigen Betätigungsstößel 30. Zu beiden Seiten daneben, bzw. den Betätigungsstößel umgebend, ist, ausgehend von der Betätigungsfläche 57, ein Entriegelungselement 83 am Betätigungskörper 82 angeformt. Es weist an seiner Oberseite eine stufenförmige Entriegelungsfläche 84 auf, in deren Verlängerung zwei Entriegelungslaschen 85 einer den Auslaßstutzen 20 umgebenden Schutzkappe 66 gegenüberliegen. Diese greifen durch Öffnungen 86 in den Betätigungsschultern 44 hindurch ins Innere der Betätigungseinheit 26 hinein und sind mittels widerhakenartigen Vorsprüngen 87 im Inneren der Betätigungsschultern an der Spendereinheit 11 festgelegt. Die Unterkante der Entriegelungslaschen 85 weist eine Einführ-Schrägfläche 88 auf.

Die Spendereinheit 11 hat, durch Flansch 42 und Ausnehmung 43, eine Druckpunkt-Schnappfunktion, wie sie anhand von Fig. 3 beschrieben wurde. Die Ausnehmungen 43 können an von der Unterseite der Betätigungsschultern hervorragenden einzelnen Stegen, die die Aufnahmehülse 40 kronenförmig umgeben, vorgesehen sein.

Die Funktion ist wie folgt: Bei der Montage werden alle Teile durch gegenseitiges Einschnappen festgelegt. Die Betätigungseinheit kann gesondert von der Spendereinheit geliefert und in Bereitschaft gehalten werden, während die Spendereinheit einschließlich des Sicherungsringes 78 gesondert aufbewahrt, aufgesetzt und später wieder abgenommen werden kann. Das Aufsetzen geschieht über das Einrasten der Rastvorsprünge 75, 76, die auch in Form einzelner Nasen am Umfang vorgesehen sein können. Fig. 12 zeigt, daß diese einrasten, jedoch der Sicherungsring 78 verhindert, daß die Betätigungsschulter mit ihrem Mantel 45 ganz an den Körper 37 bzw. die von diesem im Bereich einer umlaufenden Ausnehmung gebildeten Schultern 89 herangedrückt werden.

Die Entriegelungslasche 85 liegt mit ihrem Widerhaken 87 die Schutzkappe an der Spendereinheit 11 fest, und zwar gesichert gegen versehentliches Abnehmen. In dieser Position, die in den Figuren 12 bis 14 dargestellt ist, ist der Spender 25 gegen Betätigung gesichert. Wenn ein Benutzer auf die Betätigungsfläche 57 drückt, dann stößt die obere Stufe 90 der Entriegelungsfläche 84 auf die Entriegelungslasche 90 und verhindert, daß der Betätigungsstößel 30 die Bodenfläche 23 der Aufnahmehülse 40 erreicht bzw. einen Betätigungsdruck darauf ausüben kann.

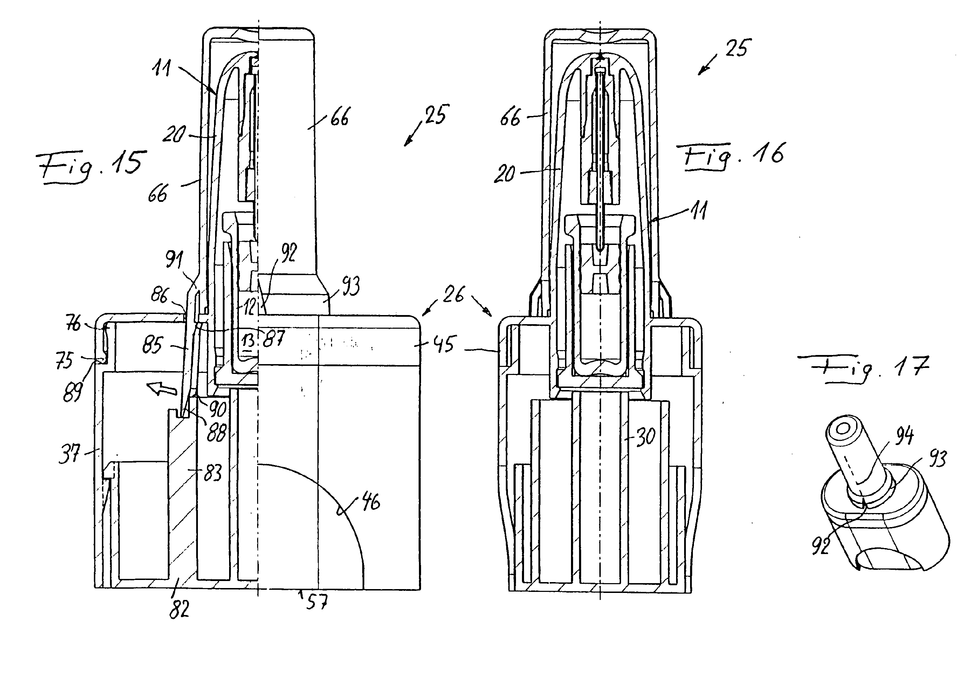

Zur Entriegelung und damit zur Vorbereitung der Betätigungsfreigabe muß der Benutzer den Sicherungsring 78 abreißen, und zwar über die Abreißlasche 79. Damit kann, wie in den Figuren 15 bis 17 dargestellt, die Spendereinheit entsprechend an die Betätigungseinheit 26 heranrücken, so daß dann die Stirnfläche des Mantels 45 auf der Schulter 89 aufliegt. Dabei drückt die Stufe 90 des Entriegelungselementes 83 über die Schrägfläche 88 die Entriegelungslasche 85 der Schutzkappe 66 nach außen, wobei sich diese um ihre Befestigungsstelle 91 am umlaufenden Mantel der Schutzkappe 66 flexibel nach außen schwenkt. In dieser Lage ist nun, wenn der Widerhaken 87 im Zusammenwirken mit der Öffnung 86 so ausgelegt ist, daß durch das Verschwenken diese voneinander frei kommen, die Schutzkappe 66 von Hand abzunehmen. Damit, d.h. erst nach Abnahme der Schutzkappe, ist der Spender betriebsfähig und kann nun durch Druck auf die Betätigungsfläche 57 in der vorher beschriebenen Weise zum Zerstäuben der in dem Medienbehälter 12 enthaltenen Flüssigkeit 13 betätigt werden, nachdem der durch die Druckpunkteinrichtung 42, 43 sichergestellte Betätigungs-Mindestdruck aufgebracht ist.

Um zusätzlich zu verhindern, daß die Betätigung erfolgt, bevor die Schutzkappe abgenommen ist, kann durch entsprechende Ausbildung des Widerhakens 87 und der Öffnung 86 aber auch vorgesehen sein, daß auch nach dem Ausschwenken der Entriegelungslaschen 85, von denen am Umfang mehrere vorgesehen sein können, die Schutzkappe noch nicht vollständig abgenommen werden kann. Nach dieser Ausführungsform wäre es notwendig, daß der Benutzer durch seitlichen Druck auf die Schutzkappe 66 diese der Länge nach aufspaltet, was durch einen Spalt 92 in einem Topfflansch 93 und eine in Fig. 17 angedeutete, durch Schwächung des Mantels der Schutzkappe 66 vorgenommene Aufbrechlinie 94 ermöglicht werden kann.

Dabei würde die Schutzkappe durch die Öffnungen und Widerhaken 86, 87 erst freigegeben werden, wenn die Schutzkappe aufgebrochen ist. Der Benutzer kann sie dann abnehmen und erst dann den Spender benutzen. Damit wird einerseits verhindert, daß der Spender bei zwar entriegelter, aber noch aufgesetzter Schutzkappe betätigt wird und andererseits wird durch Zerstörung der Schutzkappe auch signalisiert, daß bereits eine Benutzung stattgefunden hat, selbst, wenn der Benutzer die Schutzkappe nachträglich wieder auf die Spendereinheit aufsetzt, die nach der Benutzung von der Betätigungseinheit unter Überwindung der Rastvorsprünge 75, 76 abgezogen und entsorgt wird.

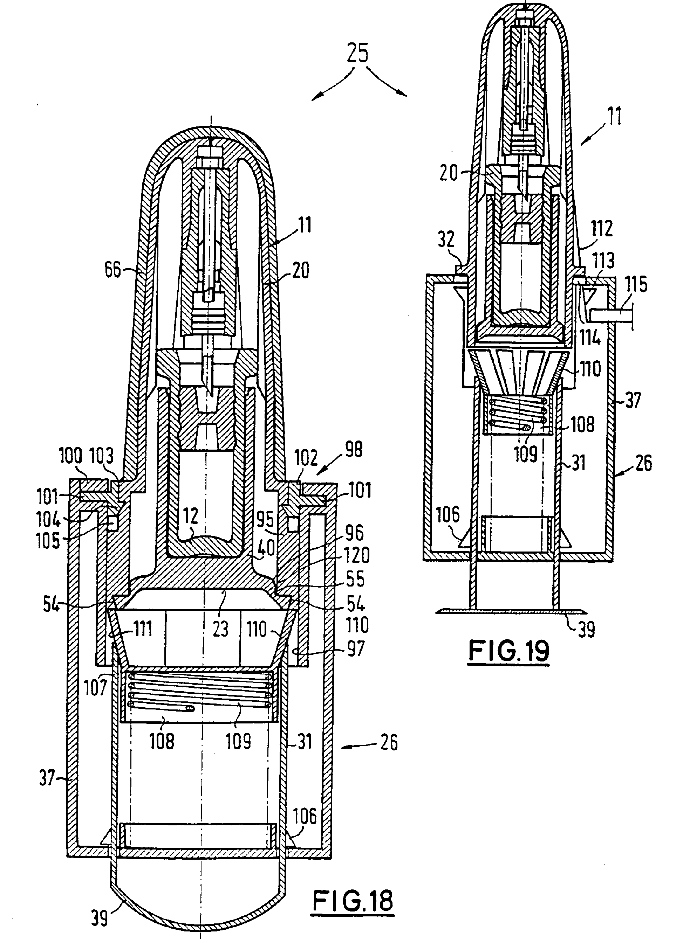

Bei dem Spender 25 nach Fig. 18 ist als Druckpunktsicherung der Spendereinheit 11 die anhand von Fig. 6 beschriebene Ausführung mit Abreißring 54 und als Sollbruchstelle dienenden Materialbrücken 55 vorgesehen. Das auch den Auslaßstutzen 20 umfassende Gehäuse der Spendereinheit 11 hat einen nach unten gerichteten rohrförmigen Vorsprung 95, in dem ein nach außen gerichteter Flansch 96 der Aufnahmehülse, an dem auch die zerstörbaren Materialbrücken vorgesehen sind, relativ dicht geführt wird und somit ein Eindringen von Schmutz ins Innere der Spendereinheit verhindert. Der dadurch entstehende enge Spalt 120 wirkt also ab Abdichtung.

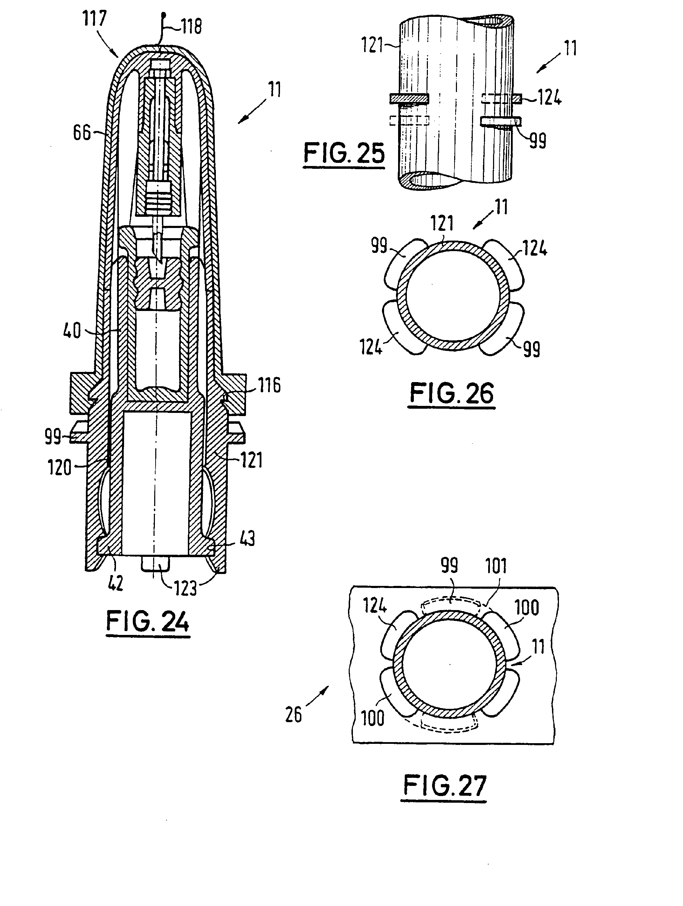

Der Vorsprung 95 ist in einen zylindrischen Rohrstutzen 97 der Betätigungseinheit 26 eingesetzt und an der Oberseite durch eine Art Kombination von Bajonett- und Schraubgewinde 98 festgelegt. Diese ist auch aus Fig. 24 zu erkennen und enthält entsprechend einer Schraubensteigung geneigte flügelartig vorspringende Rastelemente 99, die von oben her durch Ausschnitte 100 im Gehäuse 37 der Betätigungseinheit 26 eingesetzt werden und dann in gewindeartig schräggestellte Ausnehmungen 101 bis zu einem Anschlag eingedreht werden können. Diese Art der kombinierten Bajonett- und Schraubverbindung gewährleistet bei schneller und unkomplizierter Einsetzbarkeit eine feste, form- und kraftschlüssige Verbindung.

Die flügelartigen Rastelemente 99 sind an einem Ring 102 vorgesehen, der über zerstörbare Materialbrücken 103 an der Schutzkappe 66 angeformt ist. Er weist an seiner Innenseite widerhakenartige Rasten 104 auf, die in den Rohrsprung 95 der Spendereinheit eingreifen und ein Abziehen dieses Ringes nach oben verhindern. Ferner sind ggf. an der Unterseite zusammenwirkende Rastverzahnungen 105 am Ring 102 und dem Vorsprung 95 vorgesehen, um eine Verdrehung des Ringes 102 gegenüber der Spendereinheit 11 zu verhindern bzw. beide drehfest miteinander zu verbinden.

Die Betätigungseinheit 26 ist als vorspannbare und über einen Betätigungsknopf 39 auslösbare Einheit vorgesehen, wie auch anhand von Fig. 2 schon erläutert. Dazu ist im Gehäuse 37 der Betätigungseinheit 26 eine Hülse 31 vorgesehen, die unten in dem Betätigungsknopf 39 endet und die koaxial mit der Spendereinheit angeordnet und beweglich ist. Sie ist über widerhakenartige Rasten 106 im Gehäuse 37 so gehalten, daß der Betätigungsknopf 39 etwas nach unten hervorsteht, ist aber nach oben beweglich. In der Hülse 31 ist ein Auslöseelement 107 geführt, das eine napfförmige Ausnehmung 108 abgrenzt, in der eine Druckfeder 109 abgestützt ist. Diese kann sich entweder an einer unteren Gehäusewandung 37 oder im den Boden der Hülse 31 bildenden Auslöseknopf 39 abstützen. An der Oberseite des Auslöseelementes ist ein Spreizelement 110 vorgesehen, das aus einzelnen, federnden konisch nach außen gestellten Spreizlaschen besteht, die sich an der Unterseite des Ringvorsprunges 95 der Spendereinheit abstützen und durch den Rohrstutzen 97 daran gehindert sind, sich noch weiter konisch auszudehnen, da sie unter dem Druck der Druckfeder 109 stehen.

Die Hülse 31 greift auf die äußere schräge Fläche 111 des Spreizelementes.

Die Funktion ist wie folgt: Die Spendereinheit 11 wird als auswechselbares Element mit Schutzkappe 66 geliefert. Diese ist über die Rasten 104 und den Ring 102 fest mit der Spendereinheit verbunden. Beim Einsetzen der Spendereinheit in die Betätigungseinheit 26, d.h. in den Rohrstutzen 97 hinein, wirkt die untere Fläche des Ringvorsprunges 95 auf die Spreizelemente 110 und drückt diese, die vorher im Rohrstutzen weiter oben standen und ggf. auch durch eine dort vorgesehene Ringschulter verliersicher gemacht sein könnten, unter Spannung der Druckfeder 109 hinunter. Das Bajonett 98 rastet ein und wird durch Verdrehen von Spendereinheit und Betätigungseinheit gesichert und angespannt.

Dies kann auch dadurch geschehen, daß, wie beispielsweise von der unter dem Markennamen IMIGRAN im Handel befindlichen Spritze bekannt, die Betätigungseinheit entgegen der Spannkraft auf einen Aufbewahrungsbehälter für die Spendereinheit aufgedrückt wird, der einen Spannstempel hat und die Spendereinheit in einer Position festhält, die dieses Spannen und das Einsetzen durch eine Verdrehung der Betätigungseinheit ermöglicht.

Damit ist die Betätigungseinheit gespannt. Vor der Betätigung wird die Schutzkappe 66 durch Verdrehung gegenüber der Betätigungseinheit 26 und unter Abriß der Materialbrücken 103 gelöst und abgenommen. Wenn jetzt auf den Auslöseknopf 39 gedrückt wird, verschiebt sich die Hülse 31 nach oben und drückt das Spreizelement 110 zusammen, bis es an den abgeschrägten Innenseiten des Ringes 54 selbst nach innen rutscht, auf die Fläche 23 der Aufnahmehülse 40 trifft und unter dem Druck der gespannten Druckfeder 109 den Spender betätigt. Nach erfolgter Betätigung kann durch Drehung des Auslaßstutzens 12 gegenüber der Betätigungseinheit 26 die Spendereinheit 11 aus ihrer Bajonettverriegelung 98 gelöst und entnommen werden. Dabei sorgt die Verzahnung 105, die ähnlich einer HIRTH-Verzahnung ausgebildet sein kann, für die Übertragung der Drehkraft auf den Bajonettring 102.

Da hier das Einsetzen der Spendereinheit unter Federspannung erfolgt, kann es vorteilhaft sein, die Bajonett- und/oder Schraubverbindung mit einer Schnappverbindung zu verbinden, indem die Bajonett- bzw. Gewindeflankenvorsprünge 99 mit einer widerhakenartigen Schräge und entsprechender Flexibilität versehen werden, daß sie auch ohne besonders dafür vorgesehene Ausnehmungen 100 in die entsprechend ausgebildeten Gewinde- bzw. Bajonettausnehmungen 101 einrasten können. Falls sie eine Schraubkomponente enthalten, können sie dann durch weitere Verdrehung noch in ihre Endposition gebracht werden. Das Ausrasten erfolgt dabei wie bei einem Bajonett- oder Gewinde durch Drehen in Gegenrichtung und Entnehmen über einen Ausschnitt 100.

Es ist noch zu bemerken, daß der Sollbruch-Ring 54 durch entsprechende Hinterschneidung in dem Vorsprung 95 so festgelegt ist, daß die Aufnahmehülse und damit der Medienbehälter 12 nicht aus der Spendereinheit entnehmbar ist.

Fig. 19 zeigt eine Ausführung, die in Ausführung und Funktion der nach Fig. 18 entspricht. Dabei erfolgt jedoch die Festlegung der Spendereinheit 11 an der Betätigungseinheit 26 über einen federnden Rasthebel 112, der an dem Gehäuse der Spendereinheit angeformt und mit einer widerhakenartigen Rastnase 113 die Innenseite des Gehäuses 37 der Betätigungseinheit 26 hintergreift, nachdem er von oben her in die Aufnahmeöffnung 114 eingesetzt wurde. Ein Flansch 32 am Auslaßstutzen 20 bildet ein Gegenlager für die Festlegung. Zum Entnehmen der Spendereinheit nach Benutzung ist ein von dem Auslöseknopf 39 gesonderter Auswerferknopf 115 seitlich am Mantel der Betätigungseinheit vorgesehen.

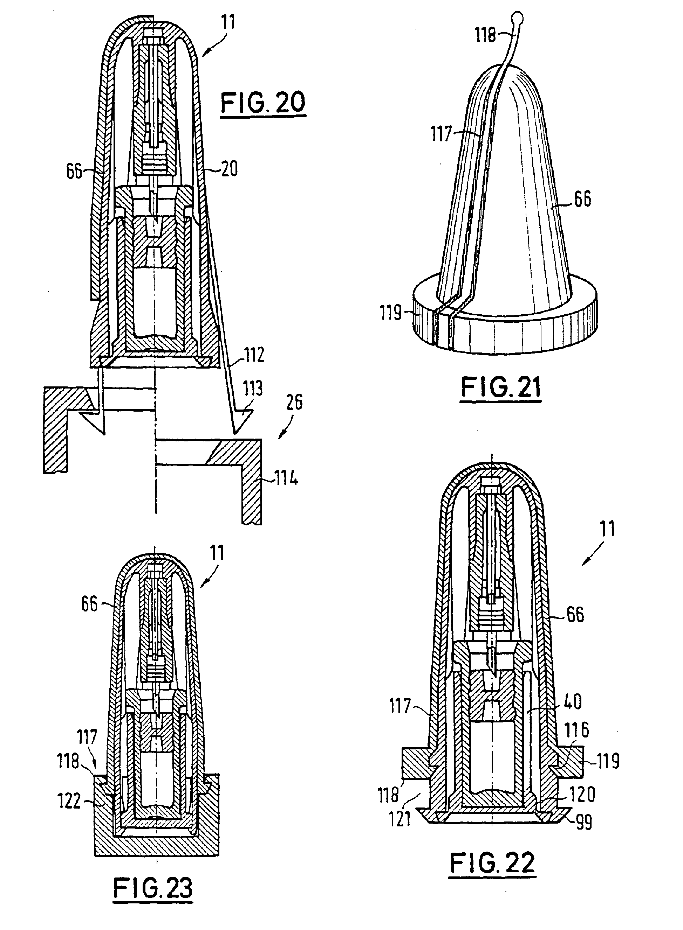

Fig. 20 zeigt eine Ausführung, bei der die Festlegung der Spendereinheit 11 an der Betätigungseinheit 26 ebenfalls über nach Art von Federbeinen gestaltete Rasthebel 112 und entsprechende widerhakenartige Rastnasen 113 erfolgt. Sie sind an der Gehäusewand der Spendereinheit 11, d.h. am Auslaßstutzen 20 so angeformt, daß sie weiter gespreizt sind als es dem Öffnungsquerschnitt der Aufnahmeöffnung 114 entspricht (Fig. 20 rechte Seite). Somit wird ein Einsetzen verhindert. Nur wenn die Schutzkappe 66 noch aufgesetzt ist (Fig. 20 linke Zeichnungsseite), drückt diese die Rasthebel 112 so weit zusammen, daß sie nun, ggf. durch eine Schräge an der Aufnahmeöffnung 114 unterstützt, in diese rastend eingreifen können.

Damit wird verhindert, daß eine Spendereinheit eingesetzt wird, bei der die Schutzkappe schon abgenommen war, die also unter Umständen schon benutzt und/oder kontaminiert ist. Die Auslösung und/oder das Auswerfen der Spendereinheit kann entsprechend Fig. 19 erfolgen.

Fig. 21 zeigt eine Art der Sicherung der Schutzkappe. Sie ist auf die Spendereinheit, wie auch aus Fig. 22 zu erkennen ist, mit einer widerartigen Hinterschnappung 116 festgelegt. Zu ihrem Abnehmen ist es notwendig, sie längs ihres Mantels aufzutrennen. Dies erfolgt über eine streifenförmige Aufreißlasche 117, die durch entsprechende linienförmige Materialschwächung von innen oder von außen, jedoch im nicht aufgerissenen Zustand dicht, mit dem Mantel verbunden ist. Eine Grifflasche 118 zum Ergreifen und Aufreißen der Schutzkappe 66 steht bei Fig. 21 am oberen geschlossenen Ende der Schutzkappe vor. Die Abreißlasche reicht bis über einen vom Mantel vorstehenden Flansch 119 (s. auch Fig. 22).

Bei Fig. 22 ist ebenfalls eine streifenförmige Aufreißlasche 117 vorgesehen, wobei dort jedoch die Grifflasche 118 im Bereich des Flansches 119 angeformt ist. Die Aufreißlasche reicht so weit über die Schutzkappe, wie dies zum einwandfreien Abnehmen nötig ist. Meist reicht schon eine Trennung im Bereich des Flansches 119, aber es ist auch möglich, die Aufreißlasche ganz über den Mantel herüberzuführen.

In Fig. 22 ist die schon anhand Fig. 18 erläuterte Einrastmechanik der Spendereinheit 11 in eine Betätigungseinheit zu erkennen, und zwar über mit einer entsprechenden Einführschräge versehene Schnapp/Bajonett/Schraub-Rastelemente 99. Auch hier ist, wie bei Fig. 18, ein sehr enger Spalt 120 zwischen der Aufnahmehülse 40 und dem Gehäuse 121 der Spendereinheit 11, die auch den Auslaßstutzen 20 beinhaltet, vorgesehen, um eine Abdichtung zu schaffen.

In Fig. 23 ist die als nachladbare Patrone ausgebildete Spendereinheit 11 im Bereich ihres Auslaßstutzens 20 im Lieferzustand von der Schutzkappe 66 überdeckt. Diese ist an der Spendereinheit dadurch festgehalten, daß sie unentnehmbar in eine Verschlußkappe 122 eingerastet ist, die die der Austrittsöffnung 19 gegenüberliegende, sonst offene Seite der Spendereinheit 11 abdeckt und gegen Schmutz sichert. Die Verschlußkappe ist nur über eine Aufreißlasche 117, 118, wie gemäß Fig. 21 bzw. 22 beschrieben, zu öffnen, so daß die Schutzkappe 66 abgenommen werden kann. Erst dann ist das Einsetzen in die Betätigungseinheit möglich.

Auch bei Fig. 24 ist eine entsprechende Aufreißlasche bzw. ein AufreiBstreifen 117, 118 vorgesehen, um die Schutzkappe abzunehmen, die im übrigen, durch eine Greifverzahnung 116, am Gehäuse 121 der Spendereinheit festgelegt ist. Fig. 24 zeigt die schon anhand Fig. 18 erläuterten, wie schräggestellte Flügel ausgebildeten Rastelemente 99 des Bajonetts 98. Sie sind jedoch am Gehäuse 121 der Spendereinheit direkt als vorspringende Segmente vorgesehen. Die Druckpunktfunktion erfolgt hier wiederum über Vorsprünge 42 an der Aufnahmehülse 40 und Ausnehmungen 43, die an kronenförmig nach unten vom Gehäuse 121 vorspringenden Stegen 123 vorgesehen sind. Es wird wiederum ein enger, möglichst staub- und schmutzsicherer Spalt 120 zwischen dem Gehäuse 121 und der Aufnahmehülse 40 vorgesehen.

Die Figuren 25 und 26 zeigen, daß die ggf. auch schraubgewindeartig schräggestellten und/oder unten zum Zwecke einer Schnappverbindung abgeschrägten Vorsprünge 99 mit entsprechenden Gegenvorsprüngen 124, die auf der Oberseite des Gehäuses 37 der Betätigungseinheit 26 aufliegen können, als in Umfangsrichtung gegeneinander versetzte Segmente ausgebildet sein können, so daß sie, wie alle Teile der beschriebenen Spender (bis auf die Nadel und eventuelle Druckfedern), leicht entformbar aus Kunststoffspritzguß herstellbar sind. Fig. 27 zeigt die Segmente 99 und 124 im Zusammenwirken mit dem Gehäuse 37. Die einander gegenüberliegenden Rastsegmente 99 können durch die entsprechenden Öffnungen 100 in die Ausnehmungen 101 eingeführt werden und durch Verdrehen in die Sicherungsposition gebracht werden. Dabei liegen die Gegensegmente 124 auf der Oberfläche des Gehäuses 37 auf.

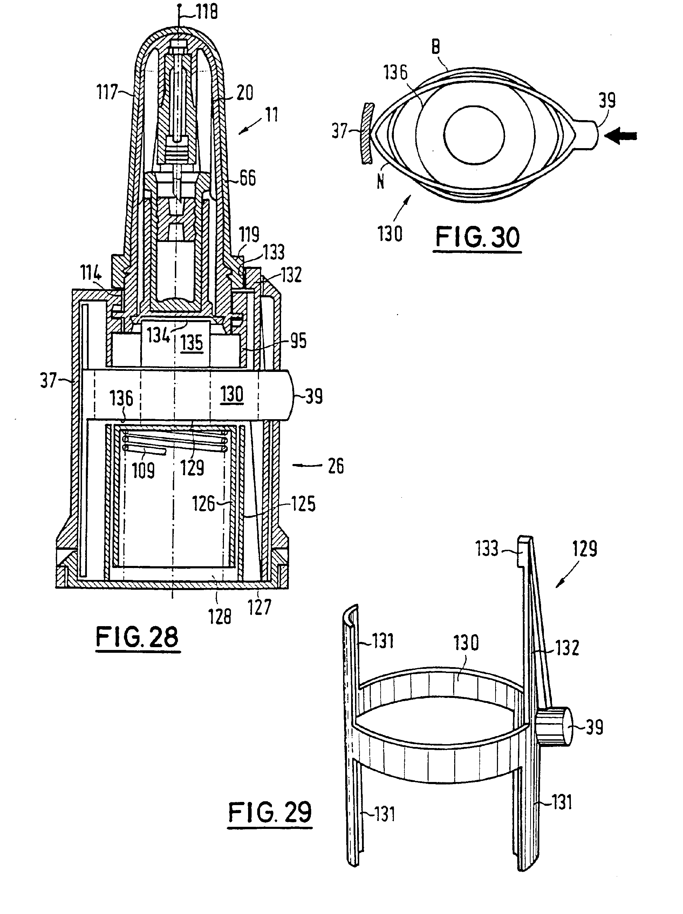

Fig. 28 zeigt eine Ausführung eines Spenders, der eine vorgespannte und auslösbare Betätigungseinrichtung 26 aufweist. Die patronenartige Spendereinheit 11 kann der nach Fig. 22 in Aufbau und Funktion entsprechen. Sie wird in das Gehäuse 37 der Betätigungseinheit 26 durch die Öffnung 114 eingesetzt und mittels Bajonett/Schraub/Schnapp-Befestigung festgelegt. Die Schraubkappe ist mittels Aufreißverschluß 117, 118 entsprechend Fig. 21 bzw. 22 an der Spendereinheit festgelegt.

Die Betätigungseinheit enthält in ihrem im wesentlichen zylindrischen Gehäuse 37 eine Druckfeder 109, die in zwei jeweils topfförmigen Führungshülsen 125, 126 eingeschlossen und geführt ist. Die Hülse 125 ist Teil eines Bodens 127, der die Betätigungseinheit 26 nach unten abschließt und durch Einrastung am Gehäusemantel 37 festgelegt ist.

Die aus Druckfeder 109 und den Führungshülsen 125, 126 bestehende Federeinheit 128 ist durch ein Auslöseelement 129 im gespannten Zustand gehalten, das in Fig. 29 perspektivisch dargestellt und in Fig. 30 in seiner Funktion erläutert ist. Das Auslöseelement besteht aus einem Kunststoffteil mit einem ovalen Ring 130, der im Normalzustand die in Fig. 30 mit "N" bezeichnete Form hat. Jeweils an den am stärksten gekrümmten Scheiteln des Ovals sind Abstützstege 131 vorgesehen, die auf der in Fig. 29 linken Rückseite nach oben und unten ragen und auf der rechten Vorderseite nur nach unten. Auf dieser Seite ist ein durch eine Gehäuseöffnung ragender Auslöseknopf 39 angeformt. Auf dieser Seite ragt nach oben ein durch Rippen versteifter Sicherungssteg 132, der mit seiner Sicherungsfläche 133, wie aus Fig. 28 zu erkennen ist, an dem Flansch 119 der Schutzkappe 66 anliegen kann.

Die Funktion ist wie folgt: Die Spendereinheit 11 wird in die Betätigungseinheit 26 eingesetzt, nachdem diese aus einem Aufbewahrungsbehälter entnommen wurde. In diesem wird sie durch ihr Einsetzen in eine dafür vorgesehene Öffnung über einen dort vorgesehenen Stempel gespannt. Dies entspricht dem bereits erwähnten IMIGRAN-System. Der von dem Stempel in dem Aufbewahrungsbehälter ausgeübte Druck wirkt auf die Fläche 134 eines Stößels 135, der von der topfförmigen Führungshülse 126 in Richtung auf die Spendereinheit hochragt, wobei er durch die Öffnung des Ringes 130 hindurchragt. Während im ausgelösten Zustand der Ring 130 durch die Führungshülse 126 etwas aufgeweitet wurde, schnappt der Ring 130 wieder in seine Ovalform N (Fig. 30) zusammen, wenn die Führungshülse 126 ihn nach unten durchquert hat, weil der Stempel 135 einen wesentlich geringeren Querschnitt als die Öffnung des Ringes 130 hat. Die sich bildende Schulter 136 stützt sich nach dem Spannen der Druckfeder 109 an der Unterseite des Ringes 130 ab, der seinerseits zwischen der Führungshülse 125 und dem Ringvorsprung 95 des Gehäuses axial unverschiebbar geführt ist. Im gespannten Zustand der Druckfeder 109 ist also der Ring 130 des Auslöseelementes 129 im entspannten Zustand. Da dies der Aufbewahrungszustand der Bedienungseinheit ist, ist dies ein Vorteil, weil sich dadurch keine Probleme mit dem Spannungsabbau durch Fließen von Kunststoff unter Belastung ergeben.

Vor der Betätigung des Spenders muß zuerst die Schutzkappe 66 entnommen werden. Sie wird über die Aufreißlasche 117, 118 aufgerissen und abgenommen. Dadurch gibt der Flansch 119, der bisher über die Sicherungsfläche 133 und den Sicherungssteg 132 eine Bewegung des Auslöseknopfes 39 weitgehend verhindert bzw. zumindest behindert hatte, diesen frei. Wenn also jetzt auf den Auslöseknopf 39 gedrückt wird, wird der ovale Ring aus seiner unbelasteten Gestalt("N" in Fig. 30) in seine betätigte Form "B" gebracht. Es ist aus Fig. 30 zu erkennen, daß die Schulter 136, die von dem Ring in Position "N" gesperrt wurde, in der Stellung "B" freigegeben ist, so daß nun die Federeinheit 128 sich entspannen kann und der Stempel 135 die Pump-Betätigung des Spenders durchführt. Diese Ausführung hat noch den Vorteil, daß sie ausschließlich mit Eigenfederung des Auslöseelementes arbeitet und daß keine engen Passungen eingehalten werden müssen. Vielmehr kann die Öffnung im Ring um Vieles größer sein als die Hülse 126, so daß ein Klemmen nicht zu befürchten ist. Sie sperrt lediglich durch die Tatsache, daß sie schon vom Spritzvorgang her entsprechend oval ausgebildet ist. Der Sicherungssteg 132 verhindert, wenn er am Flansch 119 der Schutzkappe 66 anliegt, die vollständige Verformung des Ringes in eine Form, die die Schulter freigibt. Dies muß auch nicht eine Kreisform sein, sondern kann ein schwächeres Oval sein.

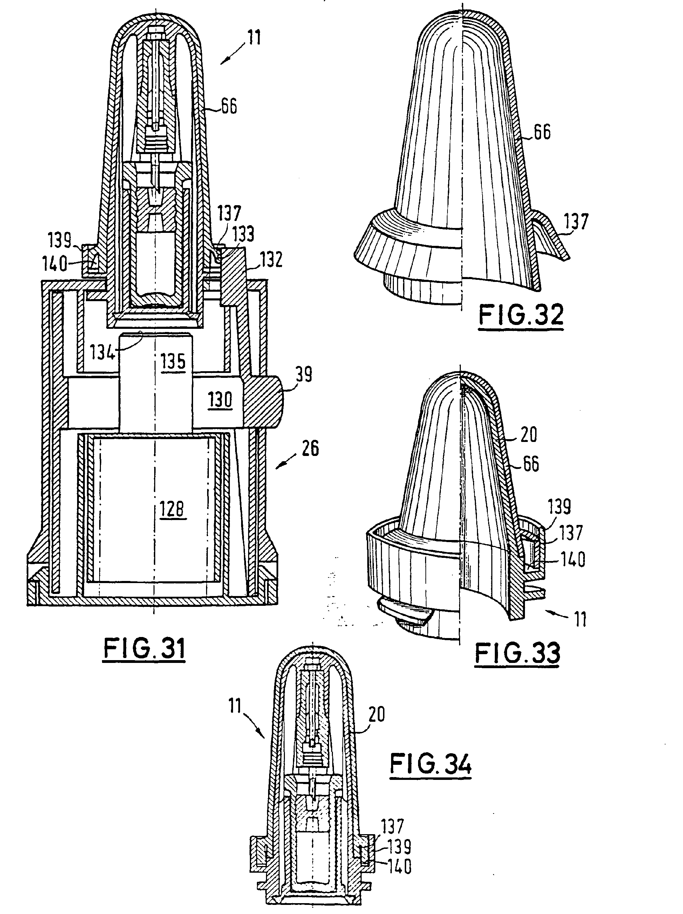

Bei einer besonders bevorzugten Ausführungsform nach Fig. 31 ist die Ausführung und Funktion entsprechend der Fig. 28 mit folgenden zusätzlichen Vorteilen: Die Schutzkappe 66 verhindert nicht nur, wie bei Fig. 28, über die Sicherungsfläche 133 und den Sicherungssteg 132 das Auslösen bei aufgesetzter Schutzkappe, sondern durch eine Verlängerung der aktiven Flächen 133 bzw. des entsprechenden Vorsprunges am oberen Ende des Sicherungsstegs 132 greift dieser auch in die Bajonett/Schraub/Schnapp-Verriegelung so ein, daß bei nicht vollständig eingerasteter Verriegelung das oder die Rastelemente 99 eine Betätigung verhindern, indem sie die entsprechende Aufweitung des Ovals bei Druck auf den Auslöseknopf 39 verhindern.

Weiter ist eine Sicherung der Schutzkappe 66 gegen unerwünschtes Wiederaufsetzen vorgesehen, wodurch versehentlich oder bewußt eine schon benutzte Spendereinheit vorgetäuscht werden würde.

Dies geschieht, wie insbesondere die Figuren 32 und 33 zeigen, dadurch, daß die Schutzkappe einen hutkrampenartig nach unten schräg ausgestellten Spreizrand 137 aufweist.

Dieser ist in Fig. 32 im Zustand bei abgezogener Schutzkappe vor dem Aufsetzen auf die Spendereinheit oder nach dem Abziehen davon. Es ist zu erkennen, daß der Spreizrand sich selbst auf einen Durchmesser aufspreizt, der ein Einsetzen in einen nach oben rinnenförmig umlaufenden Rand 139 an der Spendereinheit 11 verhindert. Das Einsetzen ist lediglich mit einer speziellen Montagevorrichtung möglich, die in der Fertigung eingesetzt wird und den Spreizrand soweit zusammendrückt, daß er in die vom Rand 139 begrenzte Rinne 140 einführbar ist.

Fig. 34 zeigt die so gesicherte Spendereinheit, die als entsprechende Nachladeinheit in dieser Form hergestellt und vom Benutzer in die Betätigungseinheit eingesetzt werden kann. Der Rand 139 hat einen entsprechenden Ausschnitt, durch den der Sicherungssteg 132 den Spreizrand 137 "abfühlt" und bei seinem Vorhandensein die Auslösung sperrt.

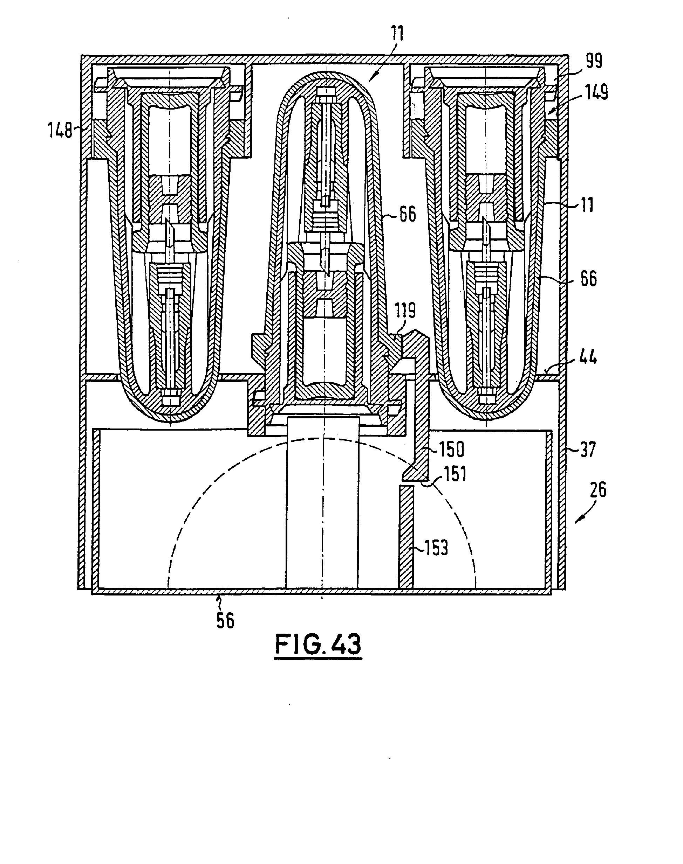

Diese Ausführung ist auch als manuell ohne Federvorspannung betätigbares System sinnvoll. In diesem Falle würde die manuelle Betätigung durch den Sicherungssteg gesperrt werden (vgl. auch Fig. 43). Das Merkmal der nicht wieder aufsetzbaren Schutzkappe 66 ist bei allen Ausführungen sinnvoll.

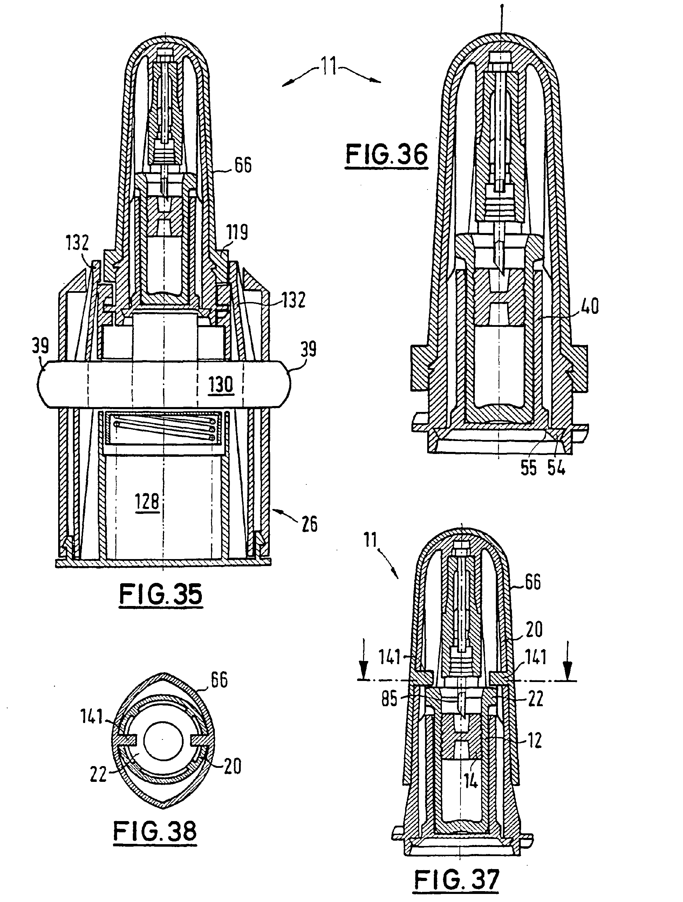

Fig. 35 zeigt eine Ausführung, die der nach Fig. 28 entspricht. Dort sind jedoch zwei einander gegenüberliegende Auslöseknöpfe 39 vorgesehen, die beide mittels eines Sicherungssteges 132 gegen Betätigung bei aufgesetzter Schutzkappe 66 gesichert sind. Auch bei dieser Ausführung läßt sich die zusätzliche Sicherung bei nicht in Sicherungsposition befindlicher Bajonett- oder Schraub- oder Schnapp-Verriegelung einsetzen.

Die Geometrie des Ovalringes 130 ist so ausgebildet, daß nur der Druck auf beide Auslöseknöpfe gleichzeitig zur Betätigung des Spenders führt. Sie bildet also eine Sicherung gegen versehentliche Betätigung (ggf. auch Kindersicherung).

Die Spendereinheit 11 nach Fig. 36 entspricht in Ausbildung und Funktion der nach Fig. 24, nur daß statt der rastenden Druckpunktfunktion der Sollbruchring 54 vorgesehen ist, der über die Materialbrücken 55 mit der Aufnahmehülse 40 verbunden ist (vgl. Fig. 6 und 18).

Fig. 37 zeigt eine Ausführung entsprechend Fig. 36, bei der jedoch die Schutzkappe 66 innere Vorsprünge oder Stifte 141 aufweist, die durch Öffnungen des Auslaßstutzens 20 hindurchragen und in die Bewegungsmechanik der Schubkolbenpumpe eingreifen. Im vorliegenden Fall greifen die Stifte 141 über den Flansch 22 des Medienbehälters 12. Er kann also auch bei einer versehentlichen Auslösung nicht oben geschoben werden und die Nadel 85 kann den Kolbenstopfen 14 nicht durchstechen. Damit wird eine Pumpenbetätigung verhindert.

Wie aus Fig. 38 zu erkennen ist, hat die Schutzkappe einen ovalen Querschnitt und ist so flexibel gestaltet, daß durch Druck quer zu den Stiften 141 die Schutzkappe eine runde oder ggf. in der anderen Richtung ovale Form annimmt, wodurch sich die Stifte aus den entsprechenden Öffnungen in dem Auslaßstutzen 20 herausziehen.

Sie geben dadurch nicht nur die Betätigungsmechanik, d.h. die Schubkolbenpumpe, frei, sondern erlauben es auch, daß die Schutzkappe nun abgenommen wird.

Ebenso wie bei den Ausführungen mit einem ovalen Auslösering 130 könnte statt eines Ovals auch eine beliebige andere Form gewählt werden, die mit der Form des mit ihr korrespondierenden Teils so zusammenwirkt, daß bei einer Verformung die entsprechende Wirkung eintritt. So könnte beispielsweise hier die Schutzkappe auch kreisrund sein, wenn der Querschnitt des Auslaßstutzens oval mit der längeren Achse in Richtung der Stifte wäre, oder es könnten ggf. schon Abflachungen an dem Auslaßstutzen im Zusammenwirken mit einem kreisrunden Schutzkappenquerschnitt ausreichen. Das gleiche gilt für den Auslösering 130, der ebenfalls eine kreisrunde oder eine andere, beispielsweise rhombische oder quadratische Form haben könnte und mit einer entsprechend ausgebildeten Schulter 136 zusammenarbeiten könnte.

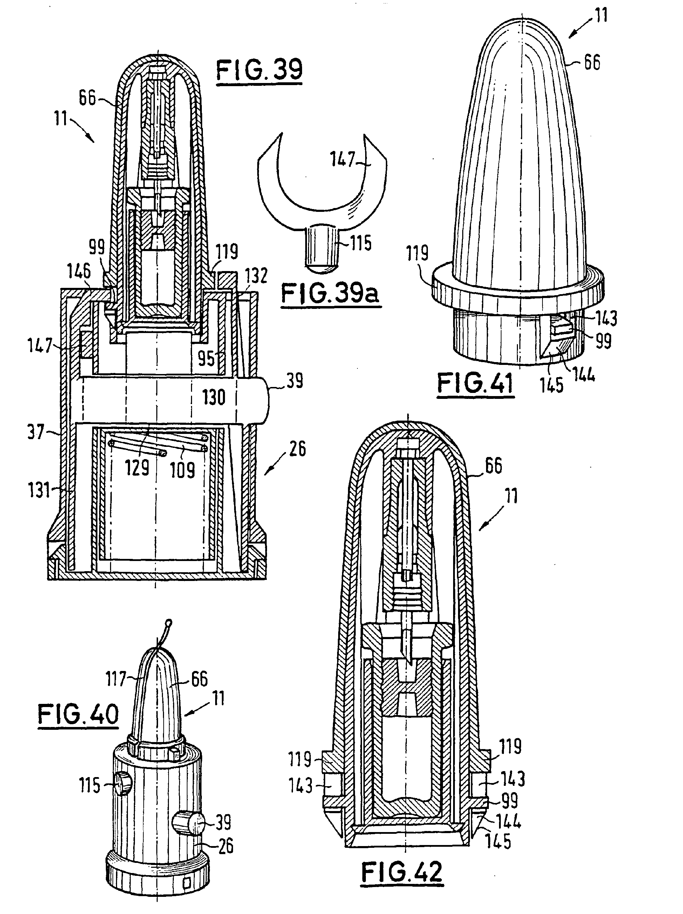

Die Figuren 39 bis 42 zeigen eine Ausführung, die bezüglich ihrer Betätigungsmechanik der nach Fig. 28 entspricht. Sie hat dementsprechend einen seitlichen Auslöseknopf 39, einen verformbaren Auslösering 130 und einen Sicherungssteg 132, der mit einem Flansch 119 der Schutzkappe 66 zusammenarbeitet.

Die in den Figuren 41 und 42 dargestellte Spendereinheit 11 hat eine Verriegelung für die Befestigung an der Betätigungseinheit 26, die mit der Befestigung der Schutzkappe 66 zusammenwirkt. An dem Flansch 119 der Schutzkappe sind z.B. an zwei einander gegenüberliegenden Stellen bügelartig nach unten ragende Elemente 143 vorgesehen, die an zwei relativ dünnen, ggf. abreißbaren Stegen 143 ein keilförmiges Verbindungsteil 144 tragen. Dieses bügelförmige Element greift jeweils über eines der Rastelemente 99, die hier als flacher Bajonett-Vorsprung ausgebildet sind, aber ebenso auch eine Schraubensteigung haben können. Die Schrägfläche 145 des Keils 144 bildet dabei eine Einführschräge, die notwendig ist, um die Spendereinheit von oben her in die entsprechende Ausnehmung der Betätigungseinheit 26 einstecken zu können. Dies geschieht unter entsprechender elastischer Verschwenkung eines Sicherungshebels 146, der anstelle eines der Abstützstege 131 aus Fig. 29 an dem Auslöseelement 129 vorgesehen ist. Damit ist sichergestellt, daß die Spendereinheit 11 nur dann eingesteckt werden kann, wenn die Schutzkappe 66 noch intakt und auf der Einheit angebracht ist. Ist die Schutzkappe durch entsprechende Drehung und Abscherung der Stege 143 entfernt, und wird dann versucht, eine solche Spendereinheit zu laden, dann ermöglicht es das Zusammenwirken des flachen Bajonett-Vorsprungs 99 mit dem Sicherungshebel 146 nicht, diesen zurückzudrücken und die Spendereinheit einzuführen.

Zum Lösen der Spendereinheit 11 von der Betätigungseinheit 26 nach dem Gebrauch dient ein Auslöseknopf 115 (s. auch Fig. 40), an dem eine an ihren beiden freien Enden keilförmige Gabel 147 angeformt ist, die in Fig. 39a gezeigt ist. Diese Gabel 147 greift, wie in Fig. 39 zu erkennen ist, zwischen den Vorsprung 95 des Gehäuses 37 und den Sicherungshebel 146 und drückt diesen nach außen, so daß die Rastelemente 99 freikommen und die Spendereinheit 11 entnommen werden kann. Die Vorsprünge 99 können sowohl zu einem Bajonett als auch einfach nur der Schnappverriegelung ohne Drehung in eine Betriebsposition entsprechen.

Nach dem Lösen der Schutzkappe, das erst erfolgen kann, wenn die Spendereinheit 11 in die Betätigungseinheit 26 eingesetzt ist, fällt der unten keilförmige Bügel frei nach unten. Es ist dann nicht mehr möglich, diese Spendereinheit wieder auf eine Betätigungseinheit aufzusetzen.

Fig. 40 zeigt eine Ausführung mit einer Aufreißlasche an der Schutzkappe 66, jedoch ist die Anordnung der Betätigungs- und Auslöseknöpfe 39 und 115 die auch für die Fig. 39 gültige.

Fig. 43 zeigt eine Betätigungseinheit 26 mit manueller Betätigung über einen Betätigungsdrücker 56, wie er auch schon in den Figuren 7 bis 9 gezeigt ist. Er ist relativ breit ausladend ausgeführt und auf ihn ist ein Deckel 148 aufsetzbar, in dem eine Reservehalterung 149 für zwei nachladbare Ersatzspendereinheiten 11 vorgesehen ist. Sie können klemmend eingesetzt oder auch über ihre Schraub-BajonettVerbindung mit propellerflügelartigen Rastelementen 99 eingesetzt sein. Mit dem Kopf ihrer Schutzkappen 66 greifen sie in entsprechende Vertiefungen in der Betätigungsschulter 44 ein, so daß sie auch bei heftigen Bewegungen in ihrer Position bleiben. Bei dieser Ausführung wird also eine andere Ausführung der schon in den Figuren 2 und 8 gezeigten Reservehalterung für "Ersatzpatronen" bzw. ihre Funktionsteile gezeigt.

An das Gehäuse 37 im Bereich der Betätigungsschultern ist ein doppelarmiger Sicherungshebel 150 angeformt, der von seiner Herstellung her so ausgebildet ist, daß er in Richtung des Pfeils 151, d.h. entgegen dem Uhrzeigersinn, schwenken möchte. Er wird daran allerdings durch den Flansch 119 der Schutzkappe 66 gehindert, wodurch er mit seiner unteren Sperrfläche 152 auf einem inneren Vorsprung 153 des Betätigungsdrückers aufsitzt und eine Betätigung verhindert, solange die Schutzkappe noch nicht abgenommen ist. Wird die Schutzkappe 66 abgenommen, schwenkt der Sicherungshebel 150 entgegen dem Uhrzeigersinn in eine die Betätigung freigebende Stellung.

## Patentansprüche

1. Spender für fließfähige Medien, insbesondere Einmal-Zerstäuber für pharmazeutische und kosmetische Flüssigkeiten,
- mit einer Spendereinheit (11), die für eine begrenzte Anzahl von Betätigungen, insbesondere ein oder zwei Betätigungen, vorgesehen ist, mit
- einem Medienbehälter (12),
- einer Austrittsöffnung (19)
- einem Austrittskanal zwischen Medienbehälter und Austrittsöffnung (19),
- einer Nasenolive, die als in die Nase eines Patienten einzuführender Stutzen ausgebildet ist,
**gekennzeichnet durch**
eine Betätigungseinheit (26), die von der Spendereinheit trennbar und wiederverwendbar ist und zur Betätigung der Spendereinheit (11) zumindest beitragende Funktionsteile aufweist, wobei die austauschbare Spendereinheit (11) alle mit dem Medium (13) und mit den zu behandelnden Körperteilen des Patienten in Kontakt kommende Bauteile enthält.

2. Spender nach Anspruch 1,
**gekennzeichnet durch**
Abschluß- und Öffnungselemente (15, 16) zwischen dem Medienbehälter (12) und der Austrittsöffnung (19),

3. Spender nach Anspruch 1 oder 2,
**gekennzeichnet durch**
eine Spendereinheit (11) mit folgenden Merkmalen:
- der Medienbehälter (12) bildet zugleich einenPumpenzylinder einer Schubkolbenpumpe;
- den Abschluß des Medienbehälters (12) bildet ein Kolbenstopfen;
- der Kolbenstopfen ist durchstechbar, insbesondere von einer Hohlnadel (16);
- ein die Hohlnadel (16) aufnehmender Kolbenstößel (18) drückt mit seiner Stirnfläche (24) auf einen Kolbenstopfen (14), um den Pumpenhub zu bewerkstelligen;
- der Kolbenstößel (18) liegt in einem Austragstutzen (20), der die Austrittsöffnung (19) aufweist.

4. Spender nach Anspruch 1 oder 2,
**dadurch gekennzeichnet, dass**
der Medienbehälter (12) zumindest teilweise innerhalb des Austragstutzens (20) liegt.

5. Spender nach einem der vorhergehenden Ansprüche, **dadurch gekennzeichnet, dass**
eine Betätigungsdruckpunkt-Einrichtung zum Sicherstellen einer Mindestbetätigungskraft vorgesehen ist, die eine federnde Schnappverbindung (42) oder zerstörbare Materialbrücken (55) aufweist.

6. Spender nach einem der vorhergehenden Ansprüche, **dadurch gekennzeichnet, dass**
zwischen einem Gehäuseteil (95) der Spendereinheit (11) und einer den Medienbehälter (12) aufnehmenden Aufnahmehülse (40) ein enger Abdichtungsspalt (120) vorgesehen ist.

7. Spender nach einem der vorhergehenden Ansprüche, **dadurch gekennzeichnet, dass**
die Betätigungseinheit (26) die Form eines vorzugsweise kreisrunden oder ovalen Stiftes hat, an dessen einem Ende die Spendereinheit (11), insbesondere über eine Ladekammer (29), einsetzbar ist.

8. Spender nach einem der vorhergehenden Ansprüche, **dadurch gekennzeichnet, dass**
in der Betätigungseinheit (26) eine Reservekammer (65) für wenigstens eine Reserve-Spendereinheit vorgesehen ist.

9. Spender nach einem der vorhergehenden Ansprüche, **dadurch gekennzeichnet, dass**
die Betätigungseinheit (26) eine Hebelbetätigung (69) mit einem Betätigungsstößel (68) enthält, die eine ggf. vorzuspannende Kunststofffeder aufweist.

10. Spender nach einem der vorhergehenden Ansprüche,
**gekennzeichnet durch**
eine Betätigungseinheit mit einer spannbaren und auslösbaren Betätigungsmechanik mit wenigstens einem der folgenden Merkmale:
- die Durchladung der Spendereinheit (11) und Spannung der Betätigungsmechanik erfolgt **durch** Drehen zweier Teile der Betätigungseinheit (26) gegeneinander;
- die Betätigungsmechanik enthält eine spannbare und einrastbare Druckfeder und eine Auslösemechanik (39);
- die Auslösemechanik enthält ein Spreizelement (110), das zur Auslösung kontraktierbar ist;
- die Auslösemechanik enthält einen formveränderlichen Ring (130), der vorzugsweise bei der Auslösung eine gegenüber einer anfänglichen Ovalform (N) in eine einem Kreis näherkommende Form (B) verformbar ist.

11. Spender nach einem der vorhergehenden Ansprüche,
**gekennzeichnet durch**
eine Betätigungssperre zum Verhindern ungewollter Betätigung des Spenders (25), die mit einem vom Einsetzen bzw. Einrasten der Spendereinheit (11) in der Betätigungseinheit (26) abhängigen Mittel (88, 89, 132, 133, 150) zusammenwirkt, mit wenigstens einem folgender Merkmale:
- die Betätigungssperre enthält einen Abreißring im Anschlußbereich zwischen Spendereinheit (21) und Betätigungseinheit (26);
- die Sperre enthält einen **durch** gegenseitige Verschiebung zwischen Spendereinheit (11) und Betätigungseinheit (26) auslenkbaren Abschnitt (85), der in der nicht ausgelenkten Stellung eine Betätigung sperrt und in der ausgelenkten Stellung diese zuläßt, wobei vorzugsweise der auslenkbare Abschnitt (85) an einer abnehmbaren Schutzkappe (66) angebracht ist und gleichzeitig eine Sicherung gegen Entnehmen der Schutzkappe vor Herstellung der Betriebsbereitschaft des Spenders (25) bildet;
- die Sperre enthält ein Sicherungselement (132, 150), das mit der Spendereinheit, vorzugsweise über eine Sicherungsfläche (133), zusammenwirkt und mit einem Auslöseelement (129) bzw. einem Betätigungselement (56) zusammenwirkt.

12. Spender nach einem der vorhergehenden Ansprüche,
**gekennzeichnet durch**
eine Betätigungssperre, die zum Verhindern der Betätigung vor Abnahme der Schutzkappe (66) mit einer Schutzkappe (66) der Spendereinheit (11) oder vor Durchführung der Verriegelung mit einer Verriegelung der Spendereinheit (11) an der Betätigungseinheit (26) zusammenarbeitet.

13. Spender nach einem der vorhergehenden Ansprüche,
**dadurch gekennzeichnet, dass**
eine zumindest die Austrittsöffnung (19) abdeckende Schutzkappe (66) durch eine Sicherung (85, 87) gegen ein Abziehen von der Spendereinheit (11) vor Herstellung der Betriebsbereitschaft des Spenders gesichert ist, und zwar durch einen auslenkbaren Abschnitt (85) mit einer Rastverbindung (86, 87), durch eine Verschlußkappe (22), die auch eine Betätigungsöffnung des Spenders (125) abschließt, wobei die Verschlußkappe vorzugsweise durch eine Aufreißlasche (117, 118) entfernbar ist, oder durch in die Spendereinheit (11) eingreifende Sicherungselemente (141), wobei diese Elemente (141) vorzugsweise durch Verformung der Schutzkappen-Querschnittsform aus dem Eingriff mit der Spendereinheit (11) entfernbar sind, wobei insbesondere Sicherungselemente (141) bilden Sicherungsanschläge, die in die Pumpenmechanik eingreifen und die Pumpe gegen Betätigung vor Abnehmen der Schutzkappe sichern.

14. Spender nach einem der vorhergehenden Ansprüche,
**dadurch gekennzeichnet, dass**
eine Schutzkappe (66), die zumindest die Austrittsöffnung (19) abdeckt, gegen unbefugtes Wiederaufsetzen durch einen Spreizring (137) gesichert ist, der montageseitig in eine rinnenartige Vertiefung (140) an der Spendereinheit (11) eingesetzt ist und nach Abnahme der Schutzkappe nicht wieder in die Rinne einfuhrbar ist.

15. Spender nach einem der vorhergehenden Ansprüche,
**dadurch gekennzeichnet, dass**
eine Verriegelung zwischender Spendereinheit (11) und der Betätigungseinheit (26) wenigstens eines der folgenden Merkmale aufweist:
- ein Bajonett, einen Schraubanschluß, durch Verschnappen, eine Rastverbindung oder federnde Andrückung über einen Anschlag;
- eine Kombination von Bajonett- und/oder Schraubanschluß, ggf. in Kombination mit einer Schnappverbindung derart, dass für das Einrasten eine Schnappverbindung vorgesehen ist, für das Lösen jedoch eine durch Verdrehen von Spendereinheit (11) und Betätigungseinheit (26) zueinander zu erreichende Löseposition vorgesehen ist;
- einen mittels eines Auslöseelementes (115) aus rastbaren Rastmechanismus (112, 113, 146).

16. Spender nach einem der vorhergehenden Ansprüche, **gekennzeichnet durch**
eine Sicherung gegen unsachgemäßes Einsetzen der Spendereinheit (11) in die Betätigungseinheit (26), bei der eine Schutzkappe (66) über der Spendereinheit Einsetz- bzw. Verriegelungselemente(112, 113) derart betätigt, dass das Einsetzen nur mit aufgesetzter Schutzkappe (66) möglich ist, vorzugsweise **durch** flexible Sicherungselemente.

17. Spender nach einem der vorhergehenden Ansprüche,
**dadurch gekennzeichnet, dass**
die Schutzkappe abreißbare Einfuhrelemente (144) aufweist, die eine Einführschräge (145) für das Einsetzen der Spendereinheit in die Betätigungseinheit (26) bilden, während die nach Entfernen der Schutzkappe (66) vorhandenen Verriegelungselemente (99) die Einführung verhindern.

## Claims

1. Dispenser for flowable media, in particular a disposable atomiser for pharmaceutical and cosmetic liquids,
- having a dispenser unit (11) which is provided for a limited number of actuations, in particular for one or two actuations, having
- a media container (12),
- an outlet opening (19)
- an outlet channel located between the media container and the outlet opening (19),
- a nose olive which is formed as a connection piece to be inserted into the nose of a patient,
**characterised by**
an actuating unit (26) that is separable from the dispenser unit and is reusable and comprises functional parts at least contributing to actuation of the dispenser unit (11), wherein the exchangeable dispenser unit (11) includes all parts coming into contact with the medium (13) and with the body parts of the patient which are to be treated.

2. Dispenser as claimed in claim 1,
**characterised by**
closing and opening elements (15, 16) between the media container (12) and the outlet opening (19).

3. Dispenser as claimed in claim 1 or 2,
**characterised by**
a dispenser unit (11) having the following features:
- the media container (12) also forms a pump cylinder of a thrust piston pump;
- the closing element of the media container (12) is formed by a piston-type stopper;
- the piston-type stopper is pierceable, in particular by a hollow needle (16);
- a piston push rod (18) receiving the hollow needle (16) presses with its end face (24) onto a piston-type stopper (14) to perform the pump stroke;
- the piston push rod (18) is located in an outlet connection piece (20) comprising the outlet opening (19).

4. Dispenser as claimed in claim 1 or 2,
**characterised in that**
the media container (12) is located at least partially within the outlet connection piece (20).

5. Dispenser as claimed in any one of the preceding claims, **characterised in that** an actuating pressure point means for ensuring a minimum actuating force is provided which comprises a resilient snap connection (42) or destructible material bridges (55).

6. Dispenser as claimed in any one of the preceding claims, **characterised in that** a narrow sealing gap (120) is provided between a casing part (95) of the dispenser unit (11) and a reception sleeve (40) receiving the media container (12).

7. Dispenser as claimed in any one of the preceding claims, **characterised in that** the actuating unit (26) is in the shape of a preferably round or oval pin, on one end of which the dispenser unit (11) can be inserted, in particular by means of a loading chamber (29).

8. Dispenser as claimed in any one of the preceding claims, **characterised in that** in the actuating unit (26) a reserve chamber (65) for at least one reserve dispenser unit is provided.

9. Dispenser as claimed in any one of the preceding claims, **characterised in that** the actuating unit (26) contains an actuating lever (69) with an actuating ram (68), which comprises a synthetic material spring which may be pretensioned.

10. Dispenser as claimed in any one of the preceding claims,
**characterised by**
an actuating unit having a tensionable and triggerable actuating mechanism having at least one of the following features:
- the loading of the dispenser unit (11) and the tensioning of the actuating mechanism is effected by turning two parts of the actuating unit (26) with respect to each other;
- the actuating mechanism contains a tensionable and latchable compression spring and a trigger mechanism (39);
- the trigger mechanism contains a spreading element (110) which can contract to effect a triggering action;
- the trigger mechanism contains a deformable ring (130) which preferably upon triggering can be deformed into a shape (B) which is more circular than an original oval shape (N).

11. Dispenser as claimed in any one of the preceding claims,
**characterised by**
an actuation blocking means for preventing undesired actuation of the dispenser (25), which cooperates with a means (88, 89, 132, 133, 150) dependent on insertion or latching of the dispenser unit (11) in the actuating unit (26), having at least one of the following features:
- the actuation blocking means contains a tear-off ring in the connection region between the dispenser unit (21) and actuating unit (26);
- the blocking means contains a portion (85) which can be deflected by mutual displacement between the dispenser unit (11) and actuating unit (26) and which in the non-deflected position blocks an actuation and in the deflected position permits such actuation, wherein preferably the deflectable portion (85) is attached to a removable protective cap (66) and at the same time forms a securing means to prevent removal of the protective cap before the dispenser (25) is in a state of operational readiness;
- the blocking means contains a securing element (132, 150) which cooperates with the dispenser unit, preferably by means of a securing surface (133), and cooperates with a triggering element (129) and/or an actuating element (56).

12. Dispenser as claimed in any one of the preceding claims,
**characterised by**
an actuation blocking means which, in order to prevent actuation, cooperates with a protective cap (66) of the dispenser unit (11) prior to removal of the protective cap (66), or cooperates with a locking arrangement of the dispenser unit (11) on the actuating unit (26) prior to locking being carried out.

13. Dispenser as claimed in any one of the preceding claims,
**characterised in that**
a protective cap (66) covering at least the outlet opening (19) is secured by a securing means (85, 87) against being pulled off the dispenser unit (11) before the dispenser is in a state of operational readiness and in particular by a deflectable portion (85) with a latching connection (86, 87) via a closure cap (22) which also closes an actuating opening of the dispenser (125), wherein the closure cap can preferably be removed by a tear-off tongue (117, 118), or by securing elements (141) engaging into the dispenser unit (11), wherein these elements (141) can preferably be removed out of engagement with the dispenser unit (11) by deformation of the protective cap cross-sectional shape, wherein in particular securing elements (141) form securing stops which engage into the pump mechanism and secure the pump against actuation prior to removal of the protective cap.

14. Dispenser as claimed in any one of the preceding claims,
**characterised in that**
a protective cap (66) which covers at least the outlet opening (19) is secured against unauthorised replacement by a spreading ring (137) which is inserted on the mounting side into a channel-like depression (140) on the dispenser unit (11) and after removal of the protective cap cannot be inserted into the channel.

15. Dispenser as claimed in any one of the preceding claims,
**characterised in that**
a locking arrangement between the dispenser unit (11) and the actuating unit (26) comprises at least one of the following features:
- a bayonet, a screw connection, by snapping, a latching connection or resilient pressing over a stop;
- a combination of bayonet and/or screw connection, possibly in combination with a snap connection such that a snap connection is provided for the latching action, however, for release purposes a release position is provided which is to be achieved by rotating the dispenser unit (11) and actuating unit (26) with respect to each other;
- a latch mechanism (112, 113, 146) which can be latched by means of a triggering element (115).

16. Dispenser as claimed in any one of the preceding claims,
**characterised by**
a means of securing against incorrect insertion of the dispenser unit (11) into the actuating unit (26), wherein a protective cap (66) over the dispenser unit actuates insertion and/or locking elements (112, 113) in such a way that insertion is possible only with the protective cap (66) in position, preferably by means of flexible securing elements.

17. Dispenser as claimed in any one of the preceding claims,
**characterised in that**
the protective cap comprises tear-off insertion elements (144) which form an insertion slope (145) for the insertion of the dispenser unit into the actuating unit (26), while the locking elements (99) which are provided after removal of the protective cap (66) prevent insertion.

## Revendications

1. Distributeur pour agents fluides, en particulier pulvérisateur pour liquides pharmaceutiques et cosmétiques,
■ avec une unité de distribution (11), qui est prévue pour un nombre limité d'actionnements, en particulier un ou deux actionnements, comprenant
■ un réservoir d'agents (12),
■ une ouverture de sortie (19)
■ un canal de sortie entre le récipient d'agents et l'ouverture de sortie (19),
■ une olive pour nez qui est conçue sous forme de tubulure à introduire dans le nez d'un patient,
**caractérisé par** une unité d'actionnement (26), qui peut être séparée de l'unité de distribution et est réutilisable et présente des parties de fonction contribuant au moins à l'actionnement de l'unité de distribution (11), l'unité de distribution (11) remplaçable contenant tous les composants venant en contact avec l'agent (13) et les parties du corps à traiter du patient.

2. Distributeur selon la revendication 1, **caractérisé par** des éléments de terminaison et d'ouverture (15, 16) entre le réservoir d'agents (12) et l'ouverture de sortie (19).

3. Distributeur selon la revendication 1 ou 2, **caractérisé par** une unité de distribution (11) présentant les caractéristiques suivantes:
■ le réservoir d'agents (12) forme en même temps un cylindre d'une pompe à piston de poussée ;
■ un bouchon de piston forme la terminaison du réservoir d'agents (12) ;
■ le bouchon de piston peut être traversé, en particulier par une aiguille creuse (16) ;
■ un coulisseau de piston (18) recevant l'aiguille creuse (16) appuie par sa surface avant (24) sur un bouchon de piston (14) afin de réaliser la course de pompe ;
■ le coulisseau de piston (18) est disposé dans une tubulure d'évacuation (20), qui présente l'ouverture de sortie (19).

4. Distributeur selon la revendication 1 ou 2, **caractérisé en ce que** le réservoir d'agents (12) est disposé au moins en partie à l'intérieur de la tubulure d'évacuation (20).

5. Distributeur selon l'une quelconque des revendications précédentes, **caractérisé en ce que** le dispositif à point de pression d'actionnement est prévu pour garantir une force d'actionnement minimum qui présente une liaison à emboitement (42) élastique ou des ponts de matériau (55) destructibles.

6. Distributeur selon l'une quelconque des revendications précédentes, **caractérisé en ce qu'**une fente d'étanchéité (120) étroite est prévue entre une partie de boîtier (95) de l'unité de distribution (11) et une douille de réception (40) recevant le récipient d'agents (12).

7. Distributeur selon l'une quelconque des revendications précédentes, **caractérisé en ce que** l'unité d'actionnement (26) a la forme d'une broche de préférence circulaire ou ovale, sur une extrémité de laquelle l'unité de distribution (11) peut être insérée, en particulier par une chambre de chargement (29).

8. Distributeur selon l'une quelconque des revendications précédentes, **caractérisé en ce qu'**une chambre de réserve (65) pour au moins une unité de distribution de réserve est prévue dans l'unité d'actionnement (26).

9. Distributeur selon l'une quelconque des revendications précédentes, **caractérisé en ce que** l'unité d'actionnement (26) contient un actionnement de levier (69) avec un coulisseau d'actionnement (68) qui présente un ressort plastique éventuellement à pré-contraindre.

10. Distributeur selon l'une quelconque des revendications précédentes, **caractérisé par** une unité d'actionnement avec un mécanisme d'actionnement pouvant être tendu et déclenché et présentant au moins l'une des caractéristiques suivantes :
■ le chargement de l'unité de distribution (11) et la tension du mécanisme d'actionnement s'effectuent par la rotation de deux parties de l'unité d'actionnement (26) l'une par rapport à l'autre ;
■ le mécanisme d'actionnement contient un ressort de pression pouvant être tendu et encliqueté et un mécanisme de déclenchement (39) ;
■ le mécanisme de déclenchement contient un élément d'écartement (110), qui peut être contracté pour le déclenchement ;
■ le mécanisme de déclenchement contient une bague (130) déformable, qui peut être déformée de préférence lors du déclenchement par rapport à une forme ovale (N) initiale en une forme (B) se rapprochant d'un cercle.

11. Distributeur selon l'une quelconque des revendications précédentes, **caractérisé par** un blocage d'actionnement pour empêcher un actionnement involontaire du distributeur (25), qui coopère avec un moyen (88, 89, 132, 133, 150) dépendant de l'insertion respectivement de l'encliquetage de l'unité de distribution (11) dans l'unité d'actionnement (26), comprenant au moins l'une des caractéristiques suivantes :
■ le blocage d'actionnement contient une bague à arracher dans la zone de raccordement entre l'unité de distribution (21) et l'unité d'actionnement (26) ;
■ le dispositif de blocage contient une partie (85) pouvant être déviée par le déplacement réciproque entre l'unité de distribution (11) et l'unité d'actionnement (26), qui bloque un actionnement dans la position non déviée et l'autorise dans la position déviée, la partie (85) déviable étant placée de préférence sur un capot de protection (66) amovible et formant en même temps une sécurité contre l'enlèvement du capot protecteur avant la préparation du bon fonctionnement du distributeur (25) ;
■ le dispositif de blocage contient un élément de sécurité (132, 150), qui coopère avec l'unité de distribution, de préférence au moyen d'une surface de sécurité (133), et coopère avec un élément de déclenchement (129) ou un élément d'actionnement (56).

12. Distributeur selon l'une quelconque des revendications précédentes, **caractérisé par** un blocage d'actionnement, qui coopère, pour empêcher l'actionnement avant l'enlèvement du capot protecteur (66), avec un capot protecteur (66) de l'unité de distribution (11) ou avant la réalisation de verrouillage avec un verrouillage de l'unité de distribution (11) sur l'unité d'actionnement (26).

13. Distributeur selon l'une quelconque des revendications précédentes, **caractérisé en ce qu'**un capot protecteur (66) recouvrant au moins l'ouverture de sortie (19) est bloqué par une sécurité (85, 87) contre un enlèvement de l'unité de distribution (11) avant l'établissement du bon fonctionnement du distributeur, et ce par une partie (85) déviable avec une liaison d'encliquetage (86, 87), par un capot de fermeture (22), qui ferme également une ouverture d'actionnement du distributeur (125), le capot de fermeture pouvant être enlevé de préférence par une patte à arracher (117, 118), ou par des éléments de sécurité (141) s'engageant dans l'unité de distribution (11), ces éléments (141) pouvant être enlevés de préférence par déformation de la forme de section du capot de protection de l'engagement avec l'unité de distribution (11), en particulier des éléments de sécurité (141) formant des butées de sécurité qui s'engagent dans le mécanisme de pompe et protègent la pompe contre l'actionnement avant l'enlèvement du capot protecteur.

14. Distributeur selon l'une quelconque des revendications précédentes, **caractérisé en ce qu'**un capot protecteur (66), qui recouvre au moins l'ouverture de sortie (19), est protégé contre une nouvelle pose non autorisée par une bague d'écartement (137), qui est insérée côté montage dans une cavité (140) du type goulotte sur l'unité de distribution (11) et ne peut pas être introduite à nouveau dans la goulotte après l'enlèvement du capot protecteur.

15. Distributeur selon l'une quelconque des revendications précédentes, **caractérisé en ce qu'**un verrouillage entre l'unité de distribution (11) et l'unité d'actionnement (26) présente au moins l'une des caractéristiques suivantes :
■ une baïonnette, un raccordement à vis, par emboîtement, une liaison à encliquetage ou un appui élastique au moyen d'une butée ;
■ une combinaison de raccordement à baïonnette et/ou de raccordement à vis, éventuellement en combinaison avec une liaison à emboitement de telle sorte qu'une liaison à emboîtement est prévue pour l'encliquetage, mais une position de détachement, qui peut être obtenue par la rotation de l'unité de distribution (11) et de l'unité d'actionnement (26) l'une par rapport à l'autre, est prévue pour le détachement ;
■ un mécanisme d'encliquetage (112, 113, 146) pouvant être désencliqueté au moyen d'un élément de déclenchement (115).

16. Distributeur selon l'une quelconque des revendications précédentes, **caractérisé par** une sécurité contre une insertion inadéquate de l'unité de distribution (11) dans l'unité d'actionnement (26), sur laquelle un capot protecteur (66) actionne au moyen de l'unité de distribution, des éléments d'insertion ou de verrouillage (112, 113) de telle sorte que l'insertion n'est possible qu'avec le capot protecteur (66) posé dessus, de préférence par des éléments de sécurité flexibles.

17. Distributeur selon l'une quelconque des revendications précédentes, **caractérisé en ce que** le capot protecteur présente des éléments d'introduction (144) pouvant être arrachés, qui forment un chanfrein d'introduction (145) pour l'insertion de l'unité de distribution dans l'unité d'actionnement (26), alors que les éléments de verrouillage (99) présents après l'enlèvement du capot protecteur (66) empêchent l'introduction.
